(19) **Europäisches Patentamt**
European Patent Office
Office européen des brevets

(11) **EP 3 582 868 B1**

(12) **EUROPÄISCHE PATENTSCHRIFT**

(45) Veröffentlichungstag und Bekanntmachung des Hinweises auf die Patenterteilung:
**07.08.2024   Patentblatt 2024/32**

(21) Anmeldenummer: **18703347.7**

(22) Anmeldetag: **12.02.2018**

(51) Internationale Patentklassifikation (IPC):
**C07C 263/20** *(2006.01)*      **B01D 1/06** *(2006.01)*
**B01D 3/00** *(2006.01)*        **B01D 19/00** *(2006.01)*
**B01D 1/00** *(2006.01)*

(52) Gemeinsame Patentklassifikation (CPC):
(C-Sets verfügbar)
**B01D 3/008; B01D 1/0064; B01D 1/06;
B01D 1/065; B01D 19/0036; B01D 19/0057;
C07C 263/20**                                    (Forts.)

(86) Internationale Anmeldenummer:
**PCT/EP2018/053397**

(87) Internationale Veröffentlichungsnummer:
**WO 2018/149776 (23.08.2018 Gazette 2018/34)**

(54) **VERTEILERVORRICHTUNG INSBESONDERE FÜR FALLFILMVERDAMPFER UND VERWENDUNG DERSELBEN**

DISTRIBUTION DEVICE, IN PARTICULAR FOR FALLING FILM EVAPORATORS AND USE OF THE SAME

DISPOSITIF DE DISTRIBUTION EN PARTICULIER POUR ÉVAPORATEUR À FILM DESCENDANT ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.02.2017   EP 17156032**

(43) Veröffentlichungstag der Anmeldung:
**25.12.2019   Patentblatt 2019/52**

(73) Patentinhaber: **Covestro Deutschland AG**
**51373 Leverkusen (DE)**

(72) Erfinder:
• **DRUMM, Christian**
**51381 Leverkusen (DE)**
• **STEFFENS, Friedhelm**
**51373 Leverkusen (DE)**

• **HOFFMANN, Dieter**
**47443 Mörs (DE)**

(74) Vertreter: **Levpat**
**c/o Covestro AG**
**Gebäude 4825**
**51365 Leverkusen (DE)**

(56) Entgegenhaltungen:
**CN-A- 103 980 159      GB-A- 1 209 119
US-A- 4 154 642        US-A- 4 199 537**

(52) Gemeinsame Patentklassifikation (CPC): (Forts.)

C-Sets
**C07C 263/20, C07C 265/14**

## Beschreibung

Die vorliegende Erfindung befasst sich mit einer Verteilervorrichtung (10000) zur gleichmäßigen Aufteilung eines Fluids (10) in 2 oder mehr Fluidströme jeweils enthaltend Gas (11) und Flüssigkeit (12), mit einem Fallfilmverdampfer (100000), in welchem die erfindungsgemäße Verteilervorrichtung zur Aufteilung der 2 oder mehr Fluidströme auf die Heizrohre des Verdampfers dient sowie mit der Verwendung der erfindungsgemäßen Verteilervorrichtung (10 000) und insbesondere des erfindungsgemäßen Fallfilmverdampfers (100000) in der Herstellung und/oder Aufarbeitung von chemischen Produkten. Die erfindungsgemäße Verteilervorrichtung ist insbesondere durch einen Drallbrecher (600) für die Gasphase (11), welche durch Auftrennung des Fluids (10) in eine Gasphase (11) und eine Flüssigphase (12) innerhalb der Verteilervorrichtung entsteht, gekennzeichnet.

Verteilervorrichtungen für Fluide, die flüssige und gasförmige, insbesondere dampfförmige Anteile enthalten oder bei denen durch Spontanverdampfung eine Gas- bzw. Dampfphase in der Verteilervorrichtung gebildet wird, sind im Stand der Technik grundsätzlich bekannt. Ein wichtiges Anwendungsgebiet für solche Verteilervorrichtungen ist ihr Einsatz in Fallfilmverdampfern. Fallfilmverdampfer finden vielfältige Anwendungen in der Industrie, vor allem bei Dampf-Flüssig-Trennverfahren wie Destillation oder Eindampfung, wobei die zur Stofftrennung erforderliche Dampfphase im Fallfilmverdampfer erzeugt wird. Neben der reinen Stofftrennung können Verdampfer auch der Durchführung von Reaktionen dienen. So offenbart beispielsweise CN 103 980 159 A die Anwendung eines Dünnschichtverdampfers in der Pyrolyse von Aminoalkylestern.

Bei Fallfilmverdampfern erfolgt die Verdampfung einer leichterflüchtigen Komponente aus einem an den beheizten Verdampferrohren herablaufenden Flüssigkeitsfilm. Eine gleichmäßige Verteilung der Flüssigkeit auf die Rohre ist dabei essenziell für den bestimmungsgemäßen Betrieb von Fallfilmverdampfern, um eine vollständige und permanente Benetzung der Rohrwand zu gewährleisten (siehe Scholl, Rinner, 2006, Verdampfung und Kondensation in Fluidverfahrenstechnik, Goedecke (Ed.), Wiley). Flüssigkeitsverteiler, die in der Haube über dem oberen Rohrboden von Fallfilmverdampfern angebracht werden und die für den spezifischen Fall ausgelegt werden, stellen dabei die gleichmäßige Verteilung sicher. Bei siedenden oder unterkühlten Flüssigkeiten soll der Verteiler die Flüssigkeit bis zum Eintritt in die Rohre dabei weitestgehend beruhigen und gleichmäßig zwischen und auf die Verdampferrohre verteilen. Bei überhitzter Flüssigkeit, kommt es in der oberen Haube über dem Rohrboden zusätzlich zu einer Entspannungsverdampfung, sodass neben der Flüssigphase noch eine Dampfphase vorliegt, die je nach Grad der Überhitzung und Druck im Verdampfer eine hohe Geschwindigkeit hat. Für diesen Fall muss der Flüssigkeitsverteiler so gestaltet werden, dass die Dampfphase nicht die Wirksamkeit der Flüssigkeitsverteilung beeinträchtigt. Dies kann z. B. erreicht werden, indem Dampf und Flüssigkeit auf dem Weg in die Verdampferrohre getrennt werden.

Insbesondere bei niedrigen Flüssigkeitsmengen pro Verdampferrohr kann der Verdampfer in einer Kreislauffahrweise derart betrieben werden, dass ein Teil der unten aus dem Verdampfer austretenden Restflüssigkeit diesem oben wieder zugeführt wird. Bei temperaturempfindlichen Produkten oder wenn Reaktionen (z. B. Polymerisationsreaktionen) in der Flüssigkeit zur Bildung von Ablagerungen führen können oder wenn Flüssigkeiten verdampft werden sollen, die von vornherein Partikel enthalten, ist im Allgemeinen jedoch ein einmaliger Durchgang ohne Umlauf erwünscht, um die Verweilzeit im Verdampfer und damit die Wahrscheinlichkeit der Bildung von Ablagerungen in den Verdampferrohren gering zu halten, was wiederum tendenziell zu niedrigeren Flüssigkeitsmengen in den Rohren führt. Je höher bei niedriger Flüssigkeitsmenge das Eindampfverhältnis ist, desto wichtiger ist es, jedes Rohr mit der gleichen Flüssigkeitsmenge zu beaufschlagen. Wenn die Rohre nicht gleichmäßig mit Flüssigkeit beaufschlagt werden, kommt es in den Rohren, die nur eine geringe Menge Flüssigkeit erhalten, zu Problemen wie zu starker Aufkonzentrierung und Trockenlaufen bis hin zur Verstopfung einzelner Rohre, während in denjenigen Rohre, die zu viel Flüssigkeit erhalten, die gewünschte Endkonzentration nicht erreicht wird. Zusätzlich kommt es bei den beschriebenen Systemen bei zu starker Beruhigung der Flüssigkeit unter Umständen zur Bildung von Ablagerungen im Bereich des Flüssigkeitsverteilers und des Rohrbodens, die schließlich zu den gleichen Problemen führen.

Neben der Sicherstellung einer möglichst gleichmäßigen Verteilung ist es auch notwendig, dass die Flüssigkeit sofort nach Eintritt in das Rohr auf der Innenwand einen stabilen Film ausbildet. Die Flüssigkeit darf nicht durch das Rohr hindurchfallen oder in Rinnsalen das Rohr hinunterlaufen, sondern sollte von Beginn an einen gleichmäßigen Film ausbilden, welcher die gesamte verfügbare Heizfläche benetzt.

Als Flüssigkeitsverteiler bei siedenden oder unterkühlten Flüssigkeiten kommen verschiedene Varianten zum Einsatz. Die gängigsten Varianten sind Lochkasten- oder Lochwannenverteiler, wobei die Flüssigkeit über mehrere Verteilerstufen (z. B. 1 bis 3 Lochplatten oder Wannen mit Überlauf an einem Zackenwehr) zwischen die Verdampferrohre auf dem Rohrboden verteilt wird (z. B. US 6,066,232, DE 29520263 U). In einer weiteren Variante läuft die Flüssigkeit aus einem Verteiler per Überlauf durch Rohre auf den glatten Rohrboden zwischen die Verdampferrohre (siehe US 3,849,232), wodurch Verstopfungen der Bohrungen und Ablagerungen auf dem Rohrboden vermieden werden können. Wenn kein Flüssigkeitsstand auf dem Rohrboden realisiert wird, ist allerdings nicht von einer guten Verteilung auf die Rohre auszugehen. Weitere gängige Varianten sehen eine Verteilung mittels Düsen (siehe z. B. CN 204159052 U, CN 203342399 U), Verteilung von einem Zentralrohr auf weitere kleine Rohre (US 4,094,734) und Verteilung über Abdeckbleche vor (DE 1126358 B, CN 203425542 U). Die gleichmäßige Verteilung wird bei diesen Varianten zum Teil durch eine Strömungsberuhigung unterstützt.

Bei Spontanverdampfung einer eintretenden überhitzten Flüssigkeit oder einem eintretenden Gas-(oder Dampf)-Flüssigkeitsgemisch sind Entspannungskammern bzw. Dampf-Flüssigkeitsabscheider beschrieben, die im einfachsten Fall als Prallplatte gestaltet sind und die eine Trennung der Phasen vorsehen, bevor die Flüssigkeit über geeignete Verteilvarianten weiterverteilt wird, die jedoch alle bei zur Bildung von Ablagerungen (sog. *Fouling*) neigenden Systemen nicht optimal sind. In DE 1126358 B werden zwei Varianten beschrieben, bei der die Dampf-Flüssig-Trennung an einer oberen Prallplatte stattfindet. Die Flüssigkeit läuft über ein Abdeckblech in einen Wannenverteiler, der die Flüssigkeit über Tropfstellen auf den Rohrboden verteilt und einen Flüssigkeitsstand in der Wanne erzeugt, um die Strömung zu beruhigen. Der Dampf wird am Wannenverteiler entweder außen vorbeigeführt und strömt von außen nach innen auf den Rohrboden und in die Rohre oder wird durch ein Zentralrohr durch die Mitte auf den Rohrboden geführt.

Bei einer weiteren Variante strömt der Dampf von der Entspannungskammer direkt in Rohre, die in die Verdampferrohre ragen. Die Flüssigkeit, die nicht mit dem Dampf mitgerissen wird, sammelt sich auf einer Lochplatte, deren Tropfstellen zwischen den Verdampferrohren angeordnet sind (CH 385169 A).

In DE 2103889 A1 ist eine Lösung beschrieben, bei der eine Entspannungsverdampfung in einem Entspannungsraum (Wannen mit Zackenwehr) stattfindet. Die Brüden strömen durch ein Zentralrohr von oben in einen Brüdensammelraum und anschließend in Rohre, die direkt auf dem Rohrboden mittig in die Verdampferrohre ragen. Die Flüssigkeit läuft über ein Zackenwehr auf den Deckel des Brüdensammelraums und gelangt von außen auf den Rohrboden, wobei kein Flüssigkeitsstand auf dem Rohrboden vorgesehen ist, sodass von keiner guten Flüssigverteilung ausgegangen werden kann.

In DE 1992031 U ist ein Verteilersystem mit einem Zulauftopf und zwei darunterliegenden Lochwannenverteilern beschrieben, wobei ein hoher Flüssigkeitsstand und eine Strömungsberuhigung gewünscht sind. Falls durch Spontanverdampfung Dampf entsteht, kann dieser im Zulauftopf nach oben entweichen und gelangt über einen äußeren Spalt zwischen unterem Wannenverteiler und Haubenwand auf den Rohrboden.

In DD 279613 A wird ein Flüssigkeits-Dampf-Gemisch in eine Trennwanne geleitet, in der sich beide Phasen trennen können. In der Trennwanne befinden sich Überlaufrohre für die Flüssigkeit, durch die die Flüssigkeit auf den Rohrboden fließt, wobei sich das Rohrende knapp über dem Rohrboden unterhalb des Flüssigkeitsspiegels befindet. Der Dampf wird wieder außen an der Trennwanne entlang geführt und strömt nach innen zu den Rohren. Bei dieser Variante wird durch die Tauchung Tropfenmitriss durch die vorbeiströmende Dampfphase vermieden.

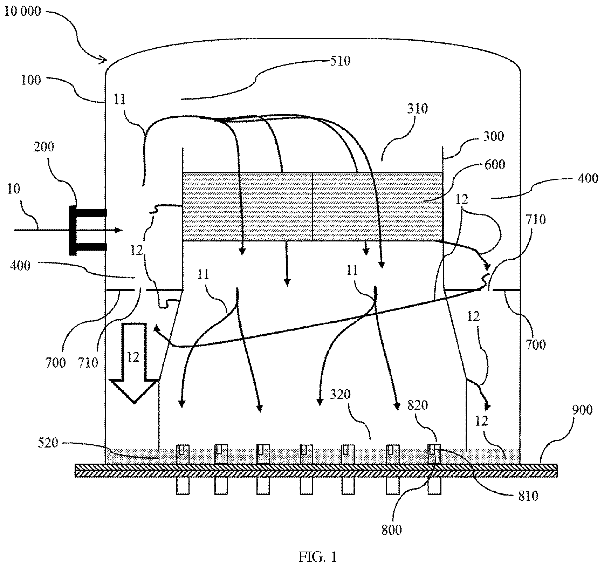

In DE 2604389 (auch veröffentlicht als US 4,154,642) ist ein tangentialer Eintritt (3 in den Zeichnungen) in einen äußeren Ringspalt (4) vorgesehen, in dem sich Dampf und Flüssigkeit trennen können. Dampf steigt nach oben und strömt dann durch einen inneren Zylinder (6), der sich nach unten hin kegelförmig aufweitet, wieder nach unten und dort direkt in die Heizrohre (2). Die Heizrohre (2) werden von einem Zylinder (6b in den Zeichnungen des US-Äquivalents), der mit dem kegelförmigen Übergangsstück verbunden ist, umfasst. Weder der Zylinder (6) noch das kegelförmige Übergangsstück verfügen über Einbauten irgendwelcher Art. Die Flüssigkeit fließt im äußeren Ringspalt (4) nach unten und durch einen Spalt (7), gebildet durch die Innenseite der Begrenzungswand des Apparats und den Zylinder (6b), auf den Rohrboden (11), wo ein hoher Flüssigkeitsstand innerhalb des Zylinders (6b) dadurch realisiert wird, dass die Heizrohre (2) weit über den Rohrboden (11) hinausragen (gut zu erkennen in FIG. 1A des US-Äquivalents, "**Lb**"). Der Einlauf der Flüssigkeit in die Heizrohre (2) erfolgt dann knapp über dem Rohrboden (11) durch Öffnungen (8) in den Rohren (2), um eine gleichmäßige Flüssigkeitsverteilung zu gewährleisten. Durch den hohen Flüssigkeitsstand und den Einlauf unter der Flüssigkeitsoberfläche sind im Verteiler gemäß DE 2604389 zwar keine Probleme bei der Flüssigkeitsverteilung zu erwarten. Allerdings ist der Verteiler wegen der zu starken Beruhigung nicht mehr für zu Ablagerungen neigende Systeme geeignet. Ein Drallbrecher für die Dampfphase wird in der Anmeldung nicht offenbart.

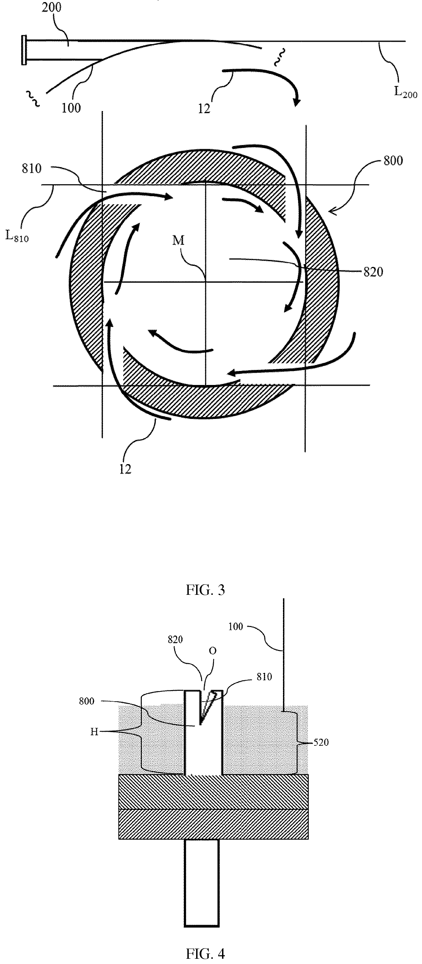

US 4,199,537 befasst sich mit dem Problem, in Rohrbündelapparaturen (z. B. Wärmeaustauschern) eine gleichmäßige Verteilung von Flüssigkeit auf der Rohrinnenseite zu gewährleisten. Zu diesem Zweck wird ein Flüssigkeitsverteiler beschrieben, bei dem die zu verteilende Flüssigkeit über einen Eintrittsstutzen (**7** oder **7'** in den Zeichnungen) durch einen Ring mit Öffnungen (6) in einen Spalt zwischen einer inneren glockenförmigen Abdeckung (5) und der Innenseite der Begrenzungswand (1) der Apparatur eingetragen wird. Durch diesen Spalt läuft die Flüssigkeit auf den Rohrboden (3), von wo aus sie durch Bohrungen in Zuführungs- bzw. Verteilerhülsen (4) in die Rohre (2) einläuft. Durch den Einlauf über die Bohrungen, mit bevorzugten Durchmessern von 2 bis 4 mm, wird auch in dieser Erfindung ein höherer Flüssigkeitsstand (27) erzeugt (siehe FIG. 4). Wie der in DE 2604389 beschriebene Verteiler neigt auch dieser Verteiler zur Bildung von Ablagerungen auf dem Rohrboden und Verstopfung der kleinen Bohrungen im Fall von Systemen, die für Fouling anfällig sind. Ein Drallbrecher für die Dampfphase wird auch in dieser Schrift nicht offenbart.

Weder in US 4,199,537 noch in der weiter oben beschriebenen DE 2604389 (auch veröffentlicht als US 4,154,642) können die Heizrohre ("**2**" in beiden Schriften) oder die Verteilerhülsen ("**4**" in US 4,199,537) als Drallbrecher für die Gasphase betrachtet werden. Verwirbelungen, Drall oder Rotationsgeschwindigkeiten in der Gasphase werden erst nach Verteilung des Fluids auf die einzelnen Rohre (2) reduziert.

Die deutsche Offenlegungsschrift DE 1 769 607 (auch veröffentlicht als GB 1 209 119) befasst sich mit einem Rohrüberlauf, welcher in einem Verdampfer mit fallendem Film die gleichmäßige Flüssigkeitsverteilung auf die Rohre sicherstellt. Der Überlauf besitzt eine rohrförmige Verlängerung, welche eine oder mehrere sich nach oben erstreckende Schlitze (14) in der Wandung und nach einwärts gerichtete Lappen (16) aufweist, so dass die Flüssigkeit bei Einlauf in tangentialer Richtung auf die Rohrinnenseite geleitet wird. Die in DE 1 769 607 beschriebenen Überläufe entsprechen den in der vorliegenden Erfindung weiter unten beschriebenen *"Führungseinrichtungen (800)"* (z. B. Rohrtüllen). Mit Hilfe von Rohrtüllen mit Schlitzen am Rohreinlauf können Höhenunterschiede des Flüssigkeitsstands auf dem Rohrboden ausgeglichen werden, ohne dass es zu massiver Fehlverteilung auf die Rohre kommt. Gleichzeitig wird eine Benetzung auf der Innenseite der Verdampferrohre sichergestellt.

In der betrieblichen Praxis hat sich gezeigt, dass die in der Literatur beschriebenen Lösungen für die gleichmäßige Flüssigkeitsverteilung gerade bei Systemen, die zur Bildung von Ablagerungen neigen, deutliche Nachteile haben. Die Bildung von Ablagerungen (auch als *Fouling* bezeichnet) kann dabei auf verschiedenste Ursachen zurückzuführen sein, z. B. feste Partikel in der zu verdampfenden Flüssigkeit, Polymerisation und Vercrackung im Verdampfer aufgrund zu langer Verweilzeit und Kristallisation (beispielsweise von gelösten Salzen) bei übersättigten Flüssigkeiten sowie unter Umständen sogar Algenbildung ("Biofouling"). Bei hohen Flüssigkeitsständen auf dem Rohrboden und in Lochwannen kommt es zu massiven Ablagerungen aufgrund zu hoher Strömungsberuhigung. Bohrungen in Lochblechen zur Flüssigkeitsverteilung und Bohrungen am Rohreinlauf neigen zu Verstopfung. Wenn der Dampf bei Auftreten von Spontanverdampfung durch Entspannung zwischen dem Verteiler und der inneren Haubenwand vorbeigeführt wird und nach innen zu den Rohren strömt, kommt es generell zu Flüssigkeitsmitriss der heruntertropfenden Flüssigkeit. Wenn die Flüssigkeit durch Rohre (sog. "Downcomer") aus dem Hauptverteiler direkt unter den Flüssigkeitsspiegel auf den Rohrboden geführt wird, um diesen Tropfenmitriss durch den vorbeiströmenden Dampf zu vermeiden, neigen auch diese Rohre am Rohraustritt knapp über dem Rohrboden zur Verstopfung (siehe oben DD 279613 A). Die Benetzung kann bei direktem Einlauf mittig in die Rohre oder glattem Rohrboden ebenfalls als nicht optimal betrachtet werden. Wenn kein Flüssigkeitsstand auf dem Rohrboden realisiert wird, um Ablagerungen zu vermeiden, ist ebenfalls von keiner guten Flüssigkeitsverteilung auszugehen.

Es bestand daher ein Bedarf an weiteren Verbesserungen bei der Ausgestaltung von Verteilervorrichtungen für Fluide, die flüssige und gasförmige (insbesondere auch dampfförmige) Anteile enthalten oder bei denen durch Entspannungsverdampfung eine Dampfphase in der Verteilervorrichtung gebildet wird. Insbesondere wäre es wünschenswert, sowohl die Bildung von Ablagerungen aufgrund zu großer Flüssigkeitsstände und Strömungsberuhigung als auch Fehlverteilungen aufgrund zu hoher Geschwindigkeiten in der Dampf- und Flüssigphase und/oder aufgrund fehlenden Flüssigkeitsstandes zu vermeiden. Weiterhin wäre es wünschenswert, Dampf und Flüssigkeit so zu trennen, dass es nicht zu Tropfenmitriss kommt und der Flüssigkeitseinlauf nicht beeinträchtigt wird.

Diesem Bedarf Rechnung tragend ist ein Gegenstand der vorliegenden Erfindung eine **Verteilervorrichtung (10000)** geeignet zum gleichmäßigen Aufteilen eines Fluids (10) in *n* Fluidströme jeweils enthaltend Gas (11) und Flüssigkeit (12), wobei *n* für eine natürliche Zahl von größer oder gleich 2, bevorzugt von 2 bis 1 000, besonders bevorzugt von 2 bis 200, ganz besonders bevorzugt von 4 bis 100 steht, wobei die Vorrichtung (10000) Folgendes umfasst:

- eine aufrecht angeordnete Haube (100), bevorzugt mit drehsymmetrischer, besonders bevorzugt runder, insbesondere kreisrunder Querschnittsfläche, die unten durch einen Boden (900) begrenzt wird;
- einen in einer seitlichen Begrenzungswand der Haube (100) angeordneten Eintrittsstutzen (200) für ein Fluid (10), welcher Eintrittsstutzen (200) so ausgestaltet ist, dass eine tangentiale Einströmung des Fluids (10) bewirkt wird;
- einen im Inneren der Haube aufrecht angeordneten Einbau (300), bevorzugt mit drehsymmetrischer, besonders bevorzugt runder, insbesondere kreisrunder Querschnittsfläche,

  ◦ der mit der Innenwand der Haube (100) einen Ringspalt (400) für nach unten strömende Flüssigkeit (12) bildet,
  ◦ dessen Oberkante oberhalb des Eintrittsstutzens (200) und unterhalb der oberen Begrenzung der Haube (100) endet, sodass die Oberkante des Einbaus (300) und die obere Begrenzung der Haube (100) einen Durchlass (510) für Gas (11) bilden,
  ◦ der oben mindestens eine Öffnung (310) für einströmendes Gas (11) aufweist,
  ◦ dessen Unterkante oberhalb des Bodens (900) endet, sodass die Unterkante des Einbaus (300) und der Boden (900) einen Durchlass (520) für Flüssigkeit (12) bilden und
  ◦ der unten mindestens eine Öffnung (320) aufweist, die mit der mindestens einen oberen Öffnung (310) strömungstechnisch verbunden ist;

- *n* Führungseinrichtungen (800) für *n* Fluidströme aus Flüssigkeit (12) und Gas (11), welche Führungseinrichtungen (800) durch die Öffnung (320) hindurch in den Einbau (300) ragen und die über Eintrittsöffnungen (810) für Flüssigkeit (12) (und zwar bevorzugt jeweils über 2 bis 10, besonders bevorzugt über 4 bis 8, Eintrittsöffnungen (810)) und über Eintrittsöffnungen (820) für Gas (11) verfügen;

wobei im Inneren des Einbaus (300) ein Drallbrecher (600) für nach unten strömendes Gas (11) angeordnet ist.

Ein weiterer Gegenstand der vorliegenden Erfindung ist ein **Fallfilmverdampfer** (100 000), bei welchem die erfindungsgemäße Verteilervorrichtung (10000) zum gleichmäßigen Aufteilen eines zu verdampfenden Fluids (10) in n Fluidströme jeweils enthaltend Gas (11) und Flüssigkeit (12) eingesetzt wird.

Ein weiterer Gegenstand der vorliegenden Erfindung ist die **Verwendung des erfindungsgemäßen Fallfilmverdampfers (100000) in der Herstellung oder Aufarbeitung eines chemischen Produkts** ausgewählt aus der Gruppe bestehend aus organischen Nitroverbindungen, primären organischen Aminen, Isocyanaten, Polyetherpolyolen und Polycarbonaten.

*"Aufrecht angeordnet"* bedeutet im Rahmen der vorliegenden Erfindung, dass der entsprechende Apparat oder das entsprechende Apparateteil im Wesentlichen lotrecht ausgerichtet ist. Bevorzugt ist selbstverständlich eine vollständig lotrechte Ausrichtung. Aufgrund fertigungsbedingter Toleranzen kann es jedoch in der Praxis zu Abweichungen von der vollständig lotrechten Ausrichtung kommen, insbesondere im Bereich von bis zu ± 3,0°, bevorzugt von bis zu ± 2,0°, besonders bevorzugt von bis zu ± 1,0°. Solche geringfügigen Abweichungen verlassen den Rahmen der vorliegenden Erfindung selbstverständlich nicht.

Eine Querschnittsfläche ist im Sinne der Erfindung *"drehsymmetrisch"*, wenn die Querschnittsfläche bei Drehung um eine durch den Flächenschwerpunkt gelegte, senkrecht zur Fläche stehende Achse um einen Winkel $\alpha$ mit sich selbst zur Deckung gebracht werden kann, wobei $\alpha$ für einen oder mehrere von 360° verschiedene(n) Winkel steht (nicht drehsymmetrisch sind daher solche Querschnittsflächen, die ausschließlich bei Drehung um 360° mit sich selbst zur Deckung gebracht werden können). Selbstredend gilt dies für jede beliebige Querschnittsfläche des betrachteten Apparats oder Apparateteils. Die einfachste (und in allen Ausführungsformen der Erfindung am stärksten bevorzugte) Möglichkeit, die erfindungsgemäße Verteilervorrichtung (10 000), den Einbau (300) und natürlich auch den erfindungsgemäßen Fallfilmverdampfer (100000) in drehsymmetrischer Form in diesem Sinne auszugestalten, ist die Konstruktion aller dieser Apparate oder Apparateteile als *Hohlzylinder.* Selbstverständlich verlassen geringfügige fertigungsbedingte Abweichungen von der idealen Zylindergeometrie nicht den Rahmen der Erfindung. Auch außen an dem jeweiligen Apparat oder Apparateteil angebrachte Einrichtungen (wie z. B. der Eintrittsstutzen (200) an der Haube (100)) gelten im Rahmen der vorliegenden Erfindung selbstverständlich nicht als Abweichung von einer drehsymmetrischen Ausgestaltung, da das Innere des jeweiligen Apparats oder Apparateteils maßgeblich ist (d. h. es wird der Innenquerschnitt des jeweiligen Hohlkörpers betrachtet).

In der erfindungsgemäßen Verteilervorrichtung (10000) findet eine Aufteilung eines *"Fluids (10)"* in *"n Fluidströme jeweils enthaltend Gas(11) und Flüssigkeit (12)"* statt. Dabei kann das Fluid (10) eine Flüssigkeit (z. B. eine in Bezug auf mindestens einen ihrer Bestandteile überhitzte Flüssigkeit) sein, die bei Eintritt in die erfindungsgemäße Verteilervorrichtung (10000) in Folge einer Druckverminderung (Entspannung) partiell verdampft (*Entspannungsverdampfung*; auch *Spontanverdampfung* genannt). In diesem Fall ist das *"Gas(11)"* der bei der partiellen Verdampfung des Fluids (10) gebildete Dampf, und die Flüssigkeit (12) ist die verbleibende, nicht in der Verteilervorrichtung (10000) verdampfte ("Rest-")Flüssigkeit. (Im Rahmen der vorliegenden Erfindung bezeichnet der Begriff *"Dampf"* die bei der Verdampfung einer Flüssigkeit gebildete Gasphase, wohingegen der Oberbegriff *"Gas"* neben dem aus der Flüssigkeit gebildeten *"Dampf"* auch weitere gasförmige Bestandteile umfassen kann.) Das Fluid (10) kann aber auch von vorneherein als ein Gas-Flüssigkeits-Gemisch (entweder weil schon vor Eintritt in die Verteilervorrichtung (10 000) ein Teil der Flüssigkeit als Dampf vorliegt oder weil ein Fremdgas wie etwa ein zur Verdünnung zugesetztes Inertgas wie Stickstoff oder ein in einem Produktgemisch gelöstes gasförmiges Neben- oder Koppelprodukt vorhanden ist) vorliegen, dessen Gasanteile in der Verteilervorrichtung (10 000) von den Flüssigkeitsanteilen getrennt werden. Selbstverständlich kann es auch in diesem Fall zusätzlich noch zu Spontanverdampfung kommen, wodurch zusätzlich zu dem von vornherein vorhandenen Dampf oder Fremdgas noch Dampf durch partielle Verdampfung *in* der Verteilervorrichtung (10000) entsteht. Ebenso ist es möglich, einen weiteren Fluidstrom (z. B. einen weiteren Gasstrom) über einen weiteren Eintrittsstutzen separat zuzuführen. In jedem Fall findet in der Verteilervorrichtung (10000) eine Trennung in eine Gasphase (11) und eine Flüssigphase (12) statt. Gas (11) und Flüssigkeit (12) durchlaufen die Verteilervorrichtung (10 000) getrennt voneinander (nämlich im Inneren des Einbaus (300) bzw. im Ringspalt (400)) und bilden so bei Eintritt in die *n* Führungseinrichtungen (800) die *"n Fluidströme enthaltend Gas (11) und Flüssigkeit (12)"* aus. Die *"n Führungseinrichtungen (800)"* im Sinne der Erfindung dienen der Aufteilung des Fluids auf n Fluidströme und sind bei Einsatz der erfindungsgemäßen Verteilervorrichtung in einem Fallfilmverdampfer mit dessen Rohren verbunden (oder sind die oberen Enden der Rohre). Die n Führungseinrichtungen (800) sind insbesondere im Boden (900) befestigt (vorzugsweiße verschweißt).

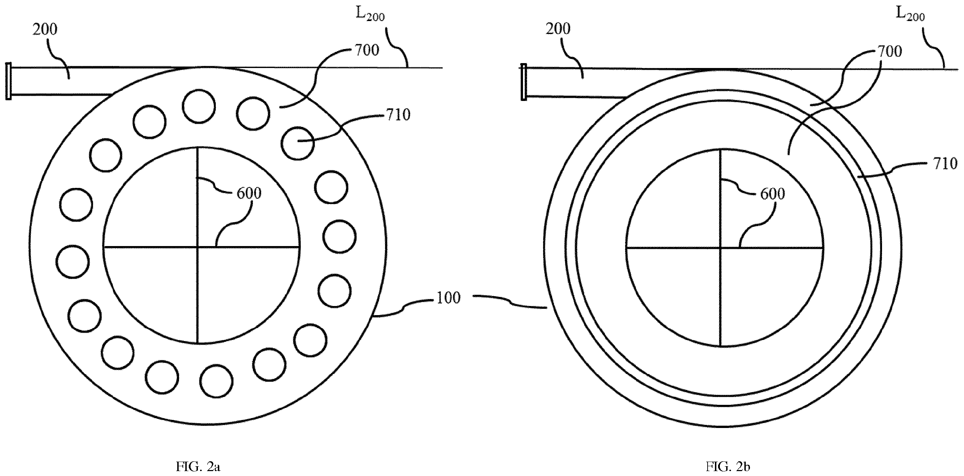

Der Eintrittsstutzen (200) (im Wesentlichen ein Hohlzylinder) ist so ausgestaltet, dass eine *"tangentiale Einströmung des Fluids (10) bewirkt wird"*, was insbesondere dann der Fall ist, wenn - in Draufsicht betrachtet - eine entlang der Längsachse des Eintrittsstutzen (200) an dessen innerer Begrenzungswand gelegte Gerade eine Tangente mit der inneren Oberfläche der Haube (100) bildet, wie in FIG 2a, b und im oberen Teil von FIG. 3 gezeigt (vgl. die Geraden $L_{200}$; die Zeichnungen sind nicht maßstabsgerecht).

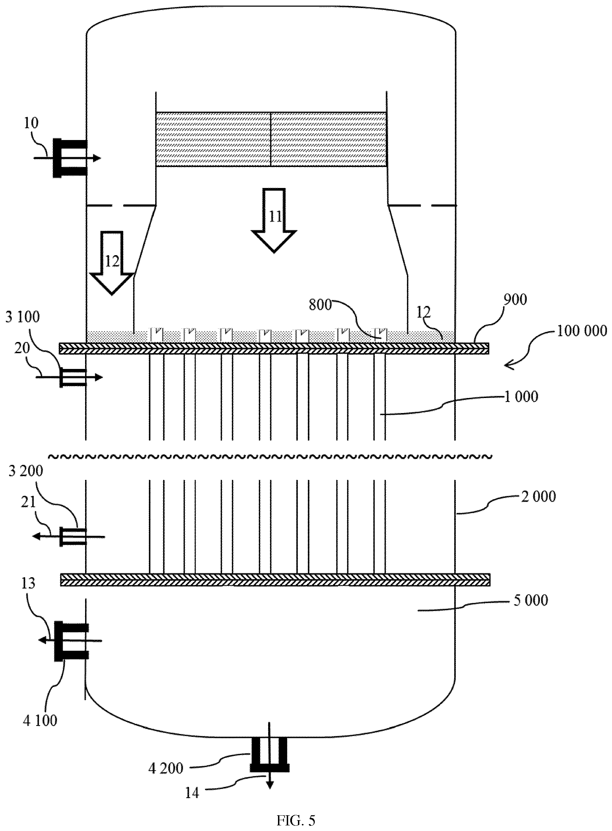

Vollkommen überraschend hat sich herausgestellt, dass z. B. der in DE 2604389 (auch veröffentlicht als US 4,154,642) beschriebene Verteiler nicht einfach dadurch für zur Bildung von Ablagerungen neigende Systeme ertüchtigt werden kann, indem man die Rohre nicht mehr so weit über den Rohrboden hinausragen lässt und anstatt eines Einlaufs über Bohrungen einen Überlauf wie in DE 1 769 607 realisiert, um den Flüssigkeitsstand auf dem Rohrboden zu verringern und so eine zu starke Beruhigung der Flüssigkeit zu vermeiden. Im beschriebenen Verteiler kommt es nämlich aufgrund des tangentialen Eintritts und der Spontanverdampfung zu hohen Geschwindigkeiten und starken Verwirbelungen in der Dampf- und Flüssigphase. Die Dampfphase bewegt sich im inneren Zylinder nicht nur nach unten auf die Rohre zu, sondern unterliegt gleichzeitig einer starken Rotationsbewegung. Gleichzeitig folgt auch die Flüssigkeit aufgrund des tangentialen Eintritts einer Rotationsbewegung im äußeren Spalt. Die Verwirbelungen in der Dampfphase führen am Rohreintritt auch zu starken Strömungen und Aufriss der Flüssigkeitsoberfläche um die Heizrohre. Die hohen Geschwindigkeiten und Verwirbelungen in Dampf und Flüssigkeit resultieren überraschenderweise in einer massiven Fehlverteilung der Flüssigkeit auf die Verdampferrohre, die mit zunehmendem Dampfanteil größer wird, da die Verwirbelungen in der Dampfphase den Flüssigkeitseinlauf stören. Diese Probleme werden durch den erfindungsgemäßen Einsatz des Drallbrechers (600) vermindert oder sogar behoben. Ein *"Drallbrecher"* im Sinne der Erfindung ist dabei zu verstehen als eine Vorrichtung, die in der Lage ist, Verwirbelungen in einer Strömung zu verhindern oder zu beseitigen, die Rotationsenergie einer Strömung zu verringern und die Strömung zu vergleichmäßigen. Im einfachsten Fall können dies zwei oder mehr Metallbleche sein, die zu einem Drallkreuz verschweißt sind und damit eine Rotationsbewegung des Fluids verhindern (siehe FIGURE 4.7 Vortex breakers prior to installion in Plant Design and Operations, Chapter 4, Piping, page 162, I. Sutton (Autor), 2017, Elsevier). Viele weitere Varianten wie Loch- und Umlenkbleche, (strukturierte) Packungen und Gestricke sind denkbar, die je nach Anordnung Verwirbelungen oder Rotationsbewegungen im Fluid beseitigen können. Der erfindungsgemäße Drallbrecher (600) für nach unten strömendes Gas ist dabei im Inneren des Einbaus (300) angeordnet, und zwar, wie in den Zeichnungen (siehe FIG. 1 und FIG. 5) gut ersichtlich, in einem Bereich desselben, in dem die nach unten strömende Flüssigkeit (12) von dem nach unten strömenden Gas (11) getrennt ist, also (signifikant) oberhalb des Durchlasses (520) für Flüssigkeit (12).

Es folgt zunächst eine Kurzzusammenfassung verschiedener möglicher **Ausführungsformen der Erfindung:**

In einer **ersten Ausführungsform der Erfindung,** die mit allen anderen Ausführungsformen kombiniert werden kann, erweitert sich der Querschnitt des Einbaus (300) nach unten hin, was in der insbesondere bevorzugten Ausgestaltung des Einbaus (300) als Körper mit kreisrunder Querschnittsfläche bedeutet, dass der Einbau (300) eine konisch-zylindrische Form hat, wie weiter unten noch näher erläutert werden wird.

In einer zweiten **Ausführungsform** der Erfindung, die ebenfalls mit allen anderen Ausführungsformen der Erfindung kombiniert werden kann, ist im Ringspalt (400) unterhalb des Eintrittsstutzens (200) ein Drallbrecher (700) *für Flüssigkeit (12)* mit Durchtrittsöffnungen (710) für die Flüssigkeit (12) angeordnet.

In einer **dritten Ausführungsform** der Erfindung, die eine besondere Ausgestaltung der zweiten Ausführungsform ist, ist der Drallbrecher (700) für Flüssigkeit (12) ein Blech, bei welchem die Durchtrittsöffnungen (710) für Flüssigkeit (12) durch mindestens zwei Löcher, die insbesondere über die gesamte Fläche des Blechs gleichmäßig verteilt sind, und/oder durch mindestens einen Schlitz, der insbesondere die gesamte Fläche des Blechs durchläuft, gebildet werden.

In einer **vierten Ausführungsform** der Erfindung, die mit allen anderen Ausführungsformen der Erfindung kombiniert werden kann, reicht die Öffnung (310) für einströmendes Gas (11) über den gesamten Innenquerschnitt des Einbaus (300).

In einer **fünften Ausführungsform** der Erfindung, die ebenfalls mit allen anderen Ausführungsformen der Erfindung kombiniert werden kann, reicht die Öffnung (320), durch welche die n Führungseinrichtungen (800) in den Einbau (300) ragen, über den gesamten Innenquerschnitt des Einbaus (300). Die vierte und fünfte Ausführungsform lassen sich am vorteilhaftesten realisieren, indem der Einbau (300) als *Hohlzylinder* ausgestaltet wird.

In einer sechsten Ausführungsform der Erfindung, die mit allen anderen Ausführungsformen der Erfindung kombiniert werden kann, sind die n Führungseinrichtungen (800) Rohrtüllen oder Bestandteile von Rohren (1000).

In einer siebten Ausführungsform der Erfindung, die eine besondere Ausgestaltung der sechsten Ausführungsform ist, sind die Eintrittsöffnungen (810) für Flüssigkeit (12)

- Tangentialschlitze, wobei die Tangentialschlitze insbesondere so angeordnet sind, dass die auf der Innenseite der Führungseinrichtungen (800) erzeugte Strömung der Flüssigkeit (12) die gleiche Richtung wie die durch die Anordnung des Eintrittsstutzens (200) im Ringspalt (400) erzeugte Strömung der Flüssigkeit (12) hat oder

- Axialschlitze.

In einer achten Ausführungsform der Erfindung, die mit allen anderen Ausführungsformen der Erfindung kombiniert werden kann, ist der Drallbrecher (600) für nach unten strömendes Gas (11) ausgewählt aus der Gruppe bestehend aus Drallkreuzen, Packungen, Drahtgestricken, Lochblechen und Umlenkblechen.

In einer **neunten Ausführungsform** der Erfindung, die mit allen anderen Ausführungsformen der Erfindung kombiniert werden kann, ragen die n Führungseinrichtungen (800) bis zu einer Höhe H im Bereich von 10 mm bis 100 mm, bevorzugt im Bereich von 20 mm bis 50 mm, in den in den Einbau (300) hinein.

**EP 3 582 868 B1**

In einer zehnten Ausführungsform der Erfindung, die mit allen anderen Ausführungsformen der Erfindung kombiniert werden kann, ist die erfindungsgemäße Verteilervorrichtung (10 000) die oberseitige Verteilervorrichtung eines Fallfilmverdampfers (100000). Der erfindungsgemäße Fallfilmverdampfer (100000) weist dabei Folgendes auf:

- einen äußeren, umhüllenden Mantel (2000),
- n Rohre (1 000), die in einem oberen und einem unteren Rohrboden befestigt sind, wobei n für eine natürliche Zahl von größer oder gleich 2, bevorzugt von 2 bis 1000, besonders bevorzugt von 2 bis 200, ganz besonders bevorzugt von 4 bis 100, steht, an deren Innenseite ein Flüssigkeitsfilm ablaufen kann,
- die erfindungsgemäße Vorrichtung (10 000) als oberseitige Verteilervorrichtung zum Verteilen von Flüssigkeit (12) und Gas (11) in die einzelnen Rohre (1 000), wobei der Boden (900) der Vorrichtung (10000) den oberen Rohrboden des Fallfilmverdampfers (100000) bildet und wobei die n Führungseinrichtungen (800) der Vorrichtung (10 000) mit den n Rohren (1 000) an deren Oberseite verbunden oder Bestandteile der n Rohre (1000) sind,
- eine Zuführeinrichtung (3 100) für ein Heizmedium (20), bevorzugt Wasserdampf, Druckwasser, Öl oder eine Salzschmelze, insbesondere Wasserdampf, in den durch die Außenseite der Rohre (1 000) und den Mantel (2000) gebildeten Rohraußenraum,
- eine Abführeinrichtung (3200) für abgekühltes Heizmedium (21), insbesondere kondensiertes Wasser, aus dem Rohraußenraum,
- eine Dampfabzugseinrichtung (4 100) für Dampf (13),
- eine Flüssigkeitsabzugseinrichtung (4200) für rohrinnenseitig nicht verdampfte Restflüssigkeit (14),
- eine Vorrichtung (5000) zum Trennen und Sammeln von Restflüssigkeit (14) und Dampf (13).

In einer **elften Ausführungsform** der Erfindung, die eine besondere Ausgestaltung der zehnten Ausführungsform ist, weist der Fallfilmverdampfer (100000) eine Rückführeinrichtung (6000) für die Rückführung eines Teils der Restflüssigkeit (14) in die Verteilervorrichtung (10000) auf, wobei diese Rückführeinrichtung (6000)

- eine Mischeinrichtung (6100) zur Vermischung des zurückgeführten Teils der Restflüssigkeit (14) mit dem Fluid (10) vor Eintritt in die Verteilervorrichtung (10000) umfasst, oder
- in einen vom Eintrittsstutzen (200) verschiedenen Eintrittsstutzen (210) mündet, welcher Eintrittsstutzen (210) in einer seitlichen Begrenzungswand der Haube (100) angeordnet ist.

In einer **zwölften Ausführungsform** der Erfindung, die mit allen anderen Ausführungsformen der Erfindung kombiniert werden kann, betrifft die Verwendung der erfindungsgemäßen Verteilervorrichtung (10 000) oder des erfindungsgemäßen Fallfilmverdampfers (100000) die Herstellung und/oder Aufarbeitung eines chemischen Produkts ausgewählt aus der Gruppe bestehend aus organischen Nitroverbindungen, organischen primären Aminen, Isocyanaten, Polyetherpolyolen und Polycarbonaten.

In einer **dreizehnten Ausführungsform** der Erfindung, die eine besondere Ausgestaltung der zwölften Ausführungsform ist, wird der Fallfilmverdampfer (100000) in der Aufarbeitung eines Isocyanats zur Verdampfung desselben eingesetzt.

In einer **vierzehnten Ausführungsform** der Erfindung, die eine besondere Ausgestaltung der dreizehnten Ausführungsform ist, wird die Verdampfung des Isocyanats zur Abreicherung desselben aus einem rückstandshaltigen Destillationssumpfstrom der Isocyanatherstellung und/oder zur Trennung einer monomeren Isocyanatfraktion, die verdampft wird, von einer polymeren Isocyanatfraktion, eingesetzt.

In einer **fünfzehnten Ausführungsform** der Erfindung, die eine besondere Ausgestaltung der dreizehnten und der vierzehnten Ausführungsform ist, wird das Isocyanat ausgewählt aus der Gruppe bestehend aus Toluylendiisocyanat, den Di- und Polyisocyanate der Diphenylmethanreihe, Hexamethylendiisocyanat, Pentamethlyendiisocyanat, Isophorondiisocyanat, Xylylendiisocyanat, Naphtalindiisocyanat und Dicyclohexylmethandiisocyanat.

Die zuvor kurz geschilderten Ausführungsformen und weitere mögliche Ausgestaltungen der Erfindung werden im Folgenden insbesondere anhand der beigefügten Zeichnungen näher erläutert. In den Zeichnungen wird die Erfindung am Beispiel eines flüssigen Fluids (10), das bei Eintritt in die Verteilervorrichtung (10000) partiell verdampft, wobei Dampf gebildet wird und nicht verdampfte Flüssigkeit verbleibt, erläutert; daher wird in der folgenden Beschreibung das Bezugszeichen 11 für Dampf und das Bezugszeichen 12 für nicht verdampfte ("Rest-")Flüssigkeit verwendet. Die Erfindung ist jedoch nicht hierauf beschränkt, sondern umfasst z. B. auch den Fall, dass das Fluid (12) von vorneherein als Gas-Flüssigkeits-Gemisch (11, 12) vorliegt oder eine Gasphase (11) separat zugeführt wird, wie weiter oben erläutert wurde. Wenn im Folgenden also von (durch partielle Verdampfung des Fluids (12) gebildetem) Dampf (11) gesprochen wird, so gilt, sofern nicht anders vermerkt, Entsprechendes stets auch für ein beliebiges Gas (11) (z. B. ein zugesetztes Fremdgas wie etwa ein zur Verdünnung zugesetztes Inertgas wie Stickstoff oder ein in einem Produktgemisch gelöstes gasförmiges Neben- oder Koppelprodukt).

Verschiedene Ausführungsformen sind, sofern sich aus dem Kontext für den Fachmann nicht eindeutig das Gegenteil ergibt, beliebig miteinander kombinierbar. Die beigefügten Abbildungen dienen der Veranschaulichung der Erfindung; sie erheben keinen Anspruch auf Maßstabstreue.

FIG. 1 zeigt eine bevorzugte Ausführungsform der erfindungsgemäßen Verteilervorrichtung (10 000). Die Verteilervorrichtung besitzt mindestens einen Eintritt (200), der ein tangentiales Einströmen des Fluids (10) ermöglicht (sog. "Tangentialstutzen"). Der tangentiale Eintritt erfolgt in einen Ringspalt (400), der durch die Innenwand der Haube (100) und die Außenwand des Einbaus (300) gebildet wird. In der Ausführungsform gemäß FIG. 1 besteht der Einbau (300) aus einem oberen Hohlzylinder und einem unteren Hohlzylinder mit größerer Querschnittsfläche, die über ein konisches Übergangsstück miteinander verbunden sind ("konisch-zylindrische Form"). Diese Ausführungsform mit sich erweiterndem Querschnitt des Einbaus (300) ist bevorzugt. In dieser Ausführungsform wird der Drallbrecher (600) für nach unten strömendes Gas im oberen zylindrischen Teil (im oberen Hohlzylinder) des Einbaus (300) angeordnet, wie in FIG. 1 und FIG. 5 gut zu sehen. Die mindestens eine obere Öffnung (310) und die mindestens eine untere Öffnung (320) entsprechen dann jeweils dem gesamten Innenquerschnitt des oberen bzw. unteren Zylinders. Dies ist die bevorzugte Ausgestaltung dieser Öffnungen. Es ist jedoch auch vorstellbar, als Einbau (300) einen einfachen Hohlzylinder mit gleichbleibendem Querschnitt zu verwenden.

Dieser Einbau (300) ragt über den Eintrittsstutzen (200), um Tropfenmitriss zu vermeiden. Im Bereich des Eintrittsstutzens (200) kommt es zur Spontanverdampfung, da das eintretende Fluid auf den Druck in der Verteilervorrichtung (10000), der geringer ist als der Druck unter dem das Fluid (10) vor Eintritt in die Verteilervorrichtung (10000) steht, entspannt wird. (Liegt das Fluid (10) von vorneherein als Gas-Flüssigkeits-Gemisch vor, kommt es einfach zu einer Trennung von Gas und Flüssigkeit wie nachfolgend beschrieben.) Da die Flüssigkeit gegen die äußere Wand (die Innenwand der Haube (100)) gedrückt wird (*tangentiale Einströmung*), kommt es im Ringspalt (400) aufgrund der Dichteunterschiede zu der gewünschten Trennung von Dampf (11) und Flüssigkeit (12). Der Dampf (11) kann ungehindert nach oben strömen und bewegt sich durch den Durchlass (510) in den Einbau (300) und durch diesen hindurch zum Boden (900), wo er durch die Öffnung (820) in die *n Führungseinrichtungen (800)* eintritt. Diese Führungseinrichtungen (800) können z. B. Rohrtüllen sein oder auch (feste) Bestandteile von Rohren (d. h. die oberen Enden von Rohren). *Rohrtüllen* können auch als *Tüllen, Verteilerhülsen* oder *Zuführungshülsen* bezeichnet werden.

Durch die bevorzugte Ausgestaltung des Einbaus (300) in konisch-zylindrischer Form mit sich nach unten erweiterndem Querschnitt wird sichergestellt, dass der Ringspalt (400) im Bereich der Spontanverdampfung breiter ist (also eine größere Querschnittsfläche verfügbar ist) als im unteren Bereich der Verteilervorrichtung, wo die Flüssigkeit (12) durch den Durchlass (den Spalt) 520 hindurchtritt und der Einbau (300) die n Führungseinrichtungen (800) umschließt. Hierdurch kann sich der Dampf (11) gleichmäßig auf die n Führungseinrichtungen (800) verteilen.

Der Einbau (300) endet mit geringem Abstand über dem Boden (900) und bildet einen Durchlass (einen *Spalt*) (520). Die Flüssigkeit (12) strömt im äußeren Ringspalt (400) nach unten (in FIG. 1 durch den Blockpfeil symbolisiert) und durch den Spalt (520) zwischen Boden (900) und der Unterkante des Einbaus (300) nach innen auf den Boden (900) und läuft durch die Öffnungen (810) in die n Führungseinrichtungen (800). Durch den Tangentialstutzen (200) und durch die spontane Verdampfung mit hohen Dampfgeschwindigkeiten wird die Flüssigkeit (12) in Rotation versetzt und folgt einer Kreisströmung im Ringspalt (400) (in FIG. 1 durch durchgehende dünne Pfeile dargestellt), die sich bis auf den Boden (900) fortpflanzt.

Eigene experimentelle Untersuchungen an Verteilervorrichtungen des Standes der Technik (ohne den Drallbrecher (600)) haben gezeigt, dass die Geschwindigkeit und Turbulenz in der Flüssigkeit (12), die in einer Kreisströmung im Ringspalt (400) auf den Boden (900) fließt, *mit zunehmender Verdampfung des Fluids (10)* (abhängig vom Grad der Überhitzung) *immer stärker werden.* Diese Kreisströmung setzt sich bis auf den Boden (900) fort. Die starke Strömung verhindert dabei die Bildung von Ablagerungen auf dem Boden (900). Des Weiteren zeigten sich in eigenen Experimenten im inneren Einbau (300) starke Verwirbelungen in der nach unten strömenden Dampfphase (11). Diese Verwirbelungen stören wiederum eine gleichmäßige Verteilung der Flüssigkeit (12) auf die n Führungseinrichtungen (800). Aufgrund der starken Strömung wird das gleichmäßige Einlaufen der Flüssigkeit in die Führungseinrichtungen (800) gestört, sodass die Flüssigkeitsmenge pro Führungseinrichtung (800) stark schwanken kann. Hierbei hat sich gezeigt, dass vor allem die äußeren Führungseinrichtungen (800) im Bereich mit der größten Turbulenz die niedrigste Flüssigkeitsmenge erhalten, während die inneren Führungseinrichtungen (800), wo es zu einer Strömungsberuhigung gekommen ist, die größten Flüssigkeitsmengen erhalten (siehe Beispiele).

Erfindungsgemäß wird ein geeigneter Drallbrecher (600) für die Dampfphase (11) im inneren Einbau (300) angebracht, um dieses Problem zu lösen. Als *Drallbrecher (600)* kann dabei, wie in FIG. 1 gezeigt, ein Drallkreuz dienen. Dieses hat bevorzugt mindestens drei Flügel (z. B. genau 3 Flügel), besonders bevorzugt mindestens vier Flügel (z. B. genau 4 Flügel, wie in FIG. 2a, b gezeigt, wo das Drallkreuz in Draufsicht zu sehen ist), außerordentlich ganz besonders bevorzugt mindestens 8 Flügel (z. B. genau 8 Flügel). Die Obergrenze der Anzahl der Flügel ist grundsätzlich nur dadurch limitiert, dass noch eine ausreichende Strömung des Dampfes (11) gewährleistet sein muss und kann vom Fachmann für einen gegebenen Fall leicht ermittelt werden. Ein solches Drallkreuz beseitigt die Kreisströmung und Verwirbelungen in der Dampfphase. Als Drallbrecher (600) kommen daneben auch Metallpackungen, Drahtgestricke, Lochbleche oder, insbesondere versetzt angeordnete, Umlenkbleche in Frage. Im eigenen Experiment konnten damit die Verwirbelungen in der Dampfphase (11) beseitigt werden, und die Flüssigkeitsverteilung wurde deutlich verbessert (siehe Beispiele).

Damit die Flüssigkeit ausgehend vom Eintrittsstutzen nicht nur an einer Stelle im unteren Ringspalt (400) auf den Flüssigkeitsspiegel auftrifft, was wiederum zu Verwirbelungen, Gasmitriss und schließlich Fehlverteilungen im Bereich des unteren Spalts (520) und der Öffnungen für den Flüssigkeitseinlauf (820) führen könnte, ist es bevorzugt, eine Vorverteilung und Vorberuhigung der Flüssigkeit im Ringspalt (400) sicherzustellen. Es ist daher bevorzugt, den Ringspalt (400) unterhalb des Eintritt Stutzens (200) mit einem Drallbrecher (700) *für Flüssigkeit (12)* zu versehen. Dieser Drallbrecher (700) für Flüssigkeit ist im einfachsten Fall ein durchgehendes Blech mit Öffnungen (710), durch die die Flüssigkeit (12) abfließen kann. Je nach Verschmutzungsneigung des Systems können diese Öffnungen durch mehrere Bohrungen (**FIG. 2a**) oder durch einen (bevorzugt durchgehenden) schmalen Schlitz (**FIG. 2b**) realisiert werden. Je nach Durchmesser und Anzahl der Bohrungen bzw. der Breite des Schlitzes kann zusätzlich ein (geringer) Flüssigkeitsstand *auf dem Drallbrecher* (700) erzeugt werden, der ebenfalls dazu beiträgt, die Strömung etwas zu beruhigen und über den gesamten Kreisumfang zu verteilen, ohne den Impuls komplett zu beseitigen. Bei starker Verschmutzungsneigung kommt dabei bevorzugt die Variante mit schmalem Schlitz (FIG. 2b) zum Einsatz, die die Strömung kaum beruhigt, sodass Ablagerungen auf dem Rohrboden vermieden werden, aber die Flüssigkeit (12) durch das Abfließen durch den Schlitz nahezu über den kompletten Kreisumfang verteilen kann. Bei geringerer Verschmutzungsneigung kann die Variante mit mehreren Bohrungen (FIG. 2a) gewählt werden, wobei die Anzahl und der Durchmesser der Bohrungen vom Fachmann leicht so berechnet werden können, dass sich ein Flüssigkeitsstand auf dem Drallbrecher (700) einstellt. Bei dieser Variante wird die Strömung je nach Flüssigkeitsstand stärker beruhigt und der Drall bzw. die Kreisströmung nahezu komplett gebrochen.

Es ist bevorzugt, den Flüssigkeitsstand auf dem Boden (900) niedrig zu halten, um Ablagerungen durch zu geringe Geschwindigkeiten der Flüssigkeit (12) zu vermeiden. Dies wird erreicht durch eine Ausgestaltung der Vorrichtung (10000) derart, dass die n Führungseinrichtungen (800) bis zu einer Höhe H (vgl. hierzu auch FIG. 4) im Bereich von 10 mm bis 100 mm, bevorzugt im Bereich von 20 mm bis 50 mm, in den Einbau (300) hineinragen.

**FIG. 3** zeigt eine Draufsicht auf eine Führungseinrichtung (800) in bevorzugter Ausführungsform. Die Führungseinrichtung (800) entspricht dabei einem aufrecht angeordneten Hohlzylinder (Rohrtülle oder (fester) Bestandteil eines Rohrs), dessen obere (offene) Seite die Eintrittsöffnung (820) für Dampf (11) ausbildet. Grundsätzlich kann man die Flüssigkeit (12) einfach über den oberen Rand des Hohlzylinders überlaufen lassen (*dann ist die* Eintrittsöffnung 820 *mit der* Eintrittsöffnung *810 identisch*); es ist jedoch bevorzugt, wie in FIG. 3 gezeigt, für den Eintritt der Flüssigkeit (12) *eigene* Eintrittsöffnungen (810) vorzusehen, die am in den Einbau (300) hineinragenden Ende der n Führungseinrichtungen (800) angeordnet sind. Bevorzugt verfügen die Führungseinrichtungen (800) jeweils über 2 bis 10, besonders bevorzugt über 4 bis 8, (eigene) Eintrittsöffnungen (810). In der in FIG. 3 gezeigten Ausführungsform sind die Eintrittsöffnungen (810) als *Tangentialschlitze* ausgeführt. Unter *Tangentialschlitzen* sind dabei solche Schlitze zu verstehen, die eine tangentiale Einströmung der Flüssigkeit (12) ermöglichen. Dies ist insbesondere dann der Fall, wenn eine entlang der Längsachse des Schlitzes an dessen innerer Begrenzungswand gelegte Gerade eine Tangente mit der inneren Oberfläche der Führungseinrichtung (800) (des Hohlzylinders) bildet, wie in FIG. 3 gezeigt (vgl. die Gerade $L_{810}$). Der Einsatz von Axialschlitzen, bei denen eine entlang der Längsachse des Schlitzes mittig durch denselben gelegte Gerade durch den Mittelpunkt M des Hohlzylinders geht, ist jedoch ebenfalls möglich.

Tangentialschlitze sind bevorzugt. Dabei ist es insbesondere bevorzugt, dass die durch die Tangentialschlitze auf der Innenseite der Führungseinrichtungen (800) erzeugte Strömung (insbesondere Kreisströmung) der Flüssigkeit (12) in gleicher Weise orientiert ist (die gleiche Richtung hat) wie die Strömung (insbesondere Kreisströmung) der Flüssigkeit (12), die durch die tangentiale Anordnung des Eintrittsstutzens (200) im Ringspalt (400) erzeugt wird. Beide Strömungen verlaufen also - von oben, wie in FIG. 3, betrachtet - jeweils entweder *im* oder *entgegen dem* Uhrzeigersinn. In FIG. 3 erzeugt der Tangentialstutzen eine Strömung im Uhrzeigersinn, die durch die korrekte Anbringung der Tangentialschlitze im Hohlzylinder (800) auch in den Führungseinrichtungen (800) fortgeführt wird. Diese Anordnung der Tangentialschlitze begünstigt ein ungehindertes Einströmen der Flüssigkeit (12) in die Eintrittsöffnungen (810) und von da in den Hohlzylinder (800), wo die Flüssigkeit (12) wie gewünscht an die Innenwand des Hohlzylinders (800) gelenkt wird und dort einen stabilen Flüssigkeitsfilm ausbildet. Bei entgegengesetzter Orientierung der Schlitze ist ein Strömungsabriss am Einlauf in den Hohlzylinder nicht auszuschließen, was die Ausbildung eines stabilen Flüssigkeitsfilm behindern würde.

Die *seitliche Form* der Eintrittsöffnungen (810) ist grundsätzlich nicht begrenzt. Neben einer rechteckigen Form ist auch eine dreieckige Form wie in **FIG. 4** dargestellt denkbar. Dazu kann der Hohlzylinder (800) z. B. einfach an dessen oberen Ende eingeschnitten und durch Umklappen eine Eintrittsöffnung (810) mit dreieckiger seitlicher Fläche gebildet werden (sog. *aufgeklappter Schlitz*).

Unabhängig von der seitlichen Form der Eintrittsöffnungen (810) ist es in jedem Fall bevorzugt, dass diese, wie in FIG. 4 beispielhaft für einen aufgeklappten Schlitz mit dreieckiger Seitenfläche gezeigt, *oben offen* sind (siehe "O" in FIG. 4).

Wie eingangs bereits erwähnt, eignet sich die erfindungsgemäße Verteilervorrichtung (10000) insbesondere für den Einsatz in einem Fallfilmverdampfer. Ein weiterer Gegenstand der Erfindung ist daher ein **Fallfilmverdampfer**

**(100000)** (vgl. auch **FIG. 5**) umfassend

- einen äußeren, umhüllenden Mantel (2000),

- *n* Rohre (1 000), die in einem oberen und einem unteren Rohrboden befestigt sind, wobei *n* für eine natürliche Zahl von größer oder gleich 2, bevorzugt von 2 bis 1000, besonders bevorzugt von 2 bis 200, ganz besonders bevorzugt von 4 bis 100, steht, an deren Innenseite ein Flüssigkeitsfilm ablaufen kann,

- eine oberseitige Verteilervorrichtung zum Verteilen von Flüssigkeit und Gas in die einzelnen Rohre (1 000),

- eine Zuführeinrichtung (3100) für ein Heizmedium (20), bevorzugt Wasserdampf, Druckwasser, Öl oder eine Salzschmelze, insbesondere Wasserdampf, in den durch die Außenseite der Rohre (1 000) und den Mantel (2000) gebildeten Rohraußenraum,

- eine Abführeinrichtung (3200) für abgekühltes Heizmedium (21), insbesondere kondensiertes Wasser, aus dem Rohraußenraum,

- eine Dampfabzugseinrichtung (4100) für Dampf (13), der sich aus dem Gas aus der oberseitigen Verteilervorrichtung und rohrinnenseitig erzeugtem Dampf zusammensetzt,

- eine Flüssigkeitsabzugseinrichtung (4200) für rohrinnenseitig nicht verdampfte Restflüssigkeit (14),

- eine, bevorzugt unterseitig angebrachte, Vorrichtung (5000) zum Trennen und Sammeln von Restflüssigkeit (14) und Dampf (13),

wobei die oberseitige Verteilervorrichtung zum Verteilen von Flüssigkeit und Gas in die einzelnen Rohre (1000) die erfindungsgemäße Vorrichtung (10000) ist, wobei der Boden (900) der Vorrichtung (10 000) den oberen Rohrboden des Fallfilmverdampfers (100000) bildet, wobei die *n* Führungseinrichtungen (800) mit den *n* Rohren (1000) an deren Oberseite verbunden sind oder wobei die *n* Führungseinrichtungen (800) Bestandteile der *n* Rohre (1000) sind.

Da die erfindungsgemäße Verteilervorrichtung (10 000) Bestandteil des erfindungsgemäßen Fallfilmverdampfers (100000) ist, ist Letzterer selbstverständlich wie Erstere ebenfalls aufrecht angeordnet und weist ebenfalls bevorzugt eine drehsymmetrische, besonders bevorzugt runde, insbesondere kreisrunde Querschnittsfläche auf. Der *"Dampf(13)"* setzt sich aus der Gasphase (11), die aus der oberseitigen Verteilervorrichtung (10 000) durch die Führungseinrichtungen (800) in die Rohre (1000) strömt, und rohrinnenseitig erzeugtem Dampf zusammen. Die Gasphase (11) besteht im einfachsten Fall aus dem durch partielle Verdampfung des Fluids (10) gebildeten Dampf. Sie kann jedoch auch, wie oben im Zusammenhang mit der erfindungsgemäßen Verteilervorrichtung (10 000) erläutert, Fremdgase enthalten oder sogar aus diesen bestehen. In den beiden letztgenannten Fällen besteht der Strom (13) streng genommen nicht nur aus Dampf; da jedoch der Hauptbestandteil des Stroms 13 rohrinnenseitig erzeugter Dampf ist, wird aus Gründen der sprachlichen Vereinfachung immer der Begriff *"Dampf"* für den Strom 13 verwendet.

Mit Ausnahme der oberseitigen Verteilervorrichtung (10000) entspricht der erfindungsgemäße Fallfilmverdampfer (100000) Fallfilmverdampfern des Standes der Technik und kann wie diese aufgebaut sein. Die Vorrichtung (5000) zum Trennen und Sammeln von Restflüssigkeit (14) und Dampf (13) kann, wie in FIG. 5 gezeigt, unmittelbar am unteren Rohrboden befestigt sein. Es ist jedoch auch möglich, dass die Rohre (1 000) in ein gemeinsames Rohrstück münden, das in die Vorrichtung (5000) übergeht, die dann beispielsweise neben dem Rohrsegment aus n Rohren (anstatt unterhalb davon) angeordnet ist.

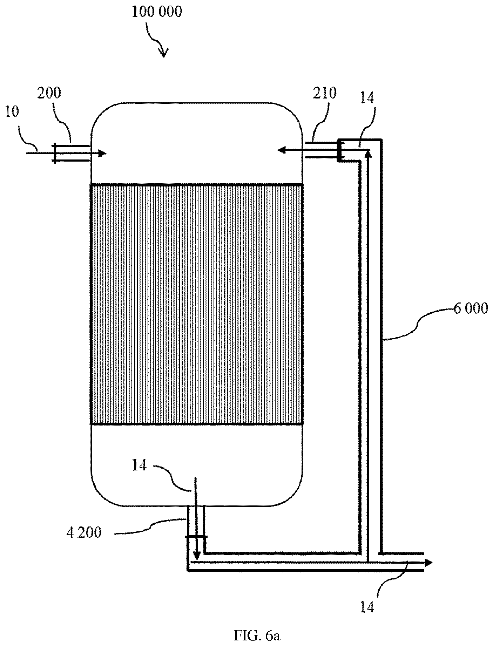

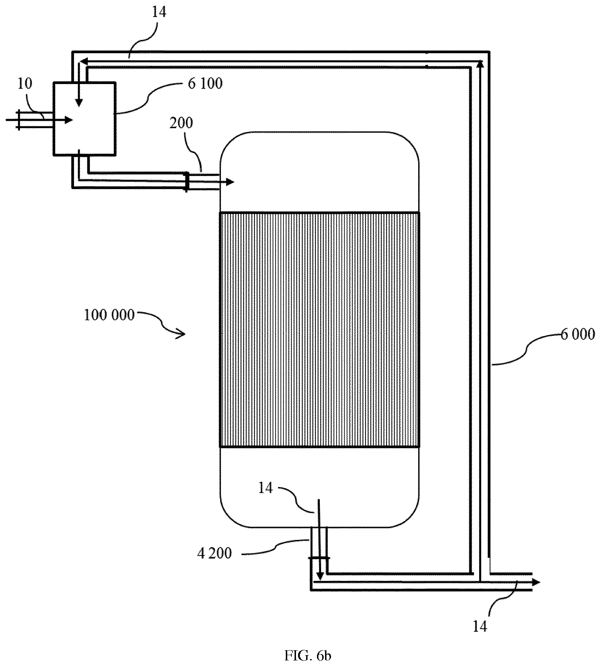

Der erfindungsgemäße Fallfilmverdampfer (100000) kann auch im Kreislauf mit einer Rückführeinrichtung (Umpumpleitung) (6000) betrieben werden (vgl. **FIG. 6a, b**; *der besseren Übersichtlichkeit halber sind viele Details aus FIG. 5 weggelassen;* ebenso nicht eingezeichnet sind Peripheriegeräte wie Pumpen). Dann wird ein Teil der unten entnommenen Restflüssigkeit (14) wieder auf den Kopf des Fallfilmverdampfers gegeben; entweder über einen separaten Eintrittsstutzen (210) (FIG. 6a) oder nach Vermischung der Restflüssigkeit (14) mit dem Feedstrom (10) in einer Mischeinrichtung (6 100); vgl. FIG. 6b. Der separate Eintrittsstutzen (210) ist dabei bevorzugt ebenfalls so angebracht, dass eine tangentiale Einströmung ermöglicht wird und ist insbesondere in der gleichen Weise orientiert wie der Eintrittsstutzen 200. Im Kreislaufbetrieb ist bevorzugt die Temperatur des Fluids (10) geringer als die Temperatur der im Kreis geführten Restflüssigkeit (14). Das vergleichsweise kalte Fluid (10) wird durch den Kontakt mit der vergleichsweise heißen Restflüssigkeit (14) so erhitzt, dass es zu Spontanverdampfung kommt.

Der erfindungsgemäße Fallfilmverdampfer kann auch als Bestandteil einer anderen Vorrichtung, beispielsweise als Verdampfer einer Destillationskolonne, eingesetzt werden.

Die Betriebsweise (insbesondere die Bedingungen von Druck und Temperatur) des erfindungsgemäßen Fallfilmverdampfers (100000) hängt von der Art des Eindampfguts ab. Der Fachmann kann hier auf die Erfahrungen des Standes der Technik zum jeweiligen Eindampfgut zurückgreifen und diese erforderlichenfalls durch einfache Routineversuche optimieren. Wenn der Fallfilmverdampfer (100000) unter gegenüber Umgebungsdruck vermindertem Druck betrieben wird, wird ein geeignetes, für den jeweiligen Anwendungsfall vom Fachmann leicht auszulegendes, Vakuumsystem eingesetzt, das bevorzugt an der Dampfabzugseinrichtung (4 100) angeschlossen wird.

Grundsätzlich ist die erfindungsgemäße Verteilervorrichtung (10 000) und insbesondere der erfindungsgemäße Fallfilmverdampfer (100000) bei beliebigen, insbesondere zur Bildung von Ablagerungen neigenden, Systemen, bei denen Flüssig- und Gasanteile (z. B. durch Spontanverdampfung) auftreten, **verwendbar.** Selbstverständlich ist ein Einsatz bei Systemen ohne besondere Tendenz zur Bildung von Ablagerungen ebenfalls möglich. Systeme mit hoher Viskosität können ebenfalls sinnvoll eingesetzt werden. Dies betrifft insbesondere Verfahren zur Herstellung und/oder Aufarbeitung von chemischen Produkten. Chemische Produkte, bei deren Herstellung und/oder Aufarbeitung die Erfindung zum Einsatz kommen kann, sind insbesondere organische Nitroverbindungen, primäre organische Amine, Isocyanate, Polyetherpolyole und Polycarbonate. Einsatzgebiete des erfindungsgemäßen Fallfilmverdampfers, allein oder ggf. in Kombination mit anderen Apparaten wie z. B. Destillationskolonnen, können z. B. Destillation (z. B. von organischen Nitroverbindungen oder von Isocyanaten), Trocknung (z. B. von organischen primären Aminen oder Polyetherpolyolen) oder Entgasen (z. B. von lösungsmittelhaltigen Polycarbonatschmelzen) sein.

Besonders bevorzugt ist der Einsatz in der Herstellung und/oder Aufarbeitung von Isocyanaten und deren Vorstufen, den entsprechenden organischen primären Aminen und organischen Nitroverbindungen. Beispiele für geeignete Isocyanate sind Toluylendiisocyanat (TDI; insbesondere *meta*-TDI), die Di- und Polyisocyanate der Diphenylmethanreihe (MDI), Hexamethylendiisocyanat (HDI), Pentamethlyendiisocyanat (PDI), Isophorondiisocyanat (IPDI), Xylylendiisocyanat (XDI, insbesondere *meta*-XDI), Naphtalindiisocyanat (NDI oder z. B. "Desmodur 15" der Covestro Deutschland AG), und Dicyclohexylmethandiisocyanat (HMDI). So umfasst etwa die Aufarbeitung von Roh-Isocyanaten regelmäßig Schritte, in denen es erforderlich ist, das zu reinigende Isocyanat zu verdampfen. Neben einer einfachen Destillation zur Abtrennung von Verunreinigungen sind hier insbesondere die Verdampfung des Isocyanats zur Abreicherung desselben aus einem rückstandshaltigen Destillationssumpfstrom der Isocyanatherstellung und/oder zur Trennung einer monomeren Isocyanatfraktion, die verdampft wird, von einer polymeren Isocyanatfraktion als sinnvolle Einsatzgebiete der Erfindung zu erwähnen.

Unabhängig von der genauen Ausgestaltung der destillativen Aufarbeitung von Roh-Isocyanaten fällt neben dem (im Allgemeinen als Destillatstrom) gewonnenen Rein-Isocyanat auch mindestens ein Destillations*sumpfstrom* an. Dieser Destillationssumpfstrom enthält den sog. Destillationsrückstand und Anteile des herzustellenden Isocyanats. Der Destillationsrückstand enthält solche Verbindungen, die unter den für die Destillation gewählten Bedingungen von Druck und Temperatur nicht verdampfen oder die sich überhaupt nicht unzersetzt verdampfen lassen. Die schwer oder gar nicht verdampfbaren Verbindungen im Destillationsrückstand sind - sofern es sich nicht um den Phosgenierungsprozess unverändert durchlaufende Verunreinigungen aus dem eingesetzten primären Amin handelt - höhermolekulare Phosgenierungsprodukte, deren Struktur nicht immer exakt bekannt ist. So kann es sich um Verbindungen handeln, die sich (formal) von Polymerisationsprodukten des eingesetzten Amins durch Ersatz der nicht polymerisierten Amindurch Isocyanat-Gruppen ableiten lassen. Der den Destillationsrückstand enthaltende Destillationssumpfstrom wird üblicherweise aufgearbeitet, wobei diese Aufarbeitung meist auch einen Schritt einer partiellen Verdampfung des in dem Destillationssumpfstrom enthaltenen herzustellenden Isocyanats umfasst, wobei ein an herzustellendem Isocyanat abgereicherter flüssiger Strom verbleibt, welcher meist noch weiter getrocknet wird. Der genannte Verdampfungsschritt kann hervorragend in dem erfindungsgemäßen Fallfilmverdampfer durchgeführt werden. Bevorzugt erfolgt diese Verdampfung bei einer Temperatur im Bereich von 120 °C bis 180 °C und bei einem Druck in der Vorrichtung (5000) zum Trennen und Sammeln von Restflüssigkeit (14) und Dampf (13) im Bereich von 20 mbar$_{(abs.)}$ bis 60 mbar$_{(abs.)}$, besonders bevorzugt bei einer Temperatur im Bereich von 130 °C bis 175 °C und bei einem Druck im Bereich von 25 mbar$_{(abs.)}$ bis 45 mbar$_{(abs.)}$. Die vorstehend beschriebene Verwendung des erfindungsgemäßen Fallfilmverdampfers ist insbesondere für die Gewinnung von TDI aus TDIhaltigen Destillationssumpfströmen geeignet.

Als Beispiel für Trennung einer monomeren Isocyanatfraktion, die verdampft wird, von einer polymeren Isocyanatfraktion als Einsatzgebiet der vorliegenden Erfindung kann die Trennung von monomerem IPDI von entsprechenden Trimeren (z. B. "Desmodur Z" der Covestro Deutschland AG) erwähnt werden.

## Beispiele

Versuche wurden für einen Flüssigverteiler im industriellen Maßstab mit einem Wasser-LuftGemisch als Testsystem durchgeführt. Die eingesetzte Testapparatur, mittels derer die Verhältnisse in einem Fallfilmverdampfer nachgestellt wurden, hat einen Durchmesser von ca. 900 mm und 45 Rohre. Um den Einfluss der Spontanverdampfung auf die Flüssigverteilung zu untersuchen, wurden in einem Fall 6 m³/h reines Wasser (*Situation ohne Spontanverdampfung*) und im anderen Fall 6 m³/h Wasser und zusätzlich 560 bzw. 1000 m³/h Luft über den tangentialen Eintrittsstutzen zugegeben (*Situation mit Spontanverdampfung*). In allen Fällen wurden die Flüssigkeitsvolumenströme pro Rohr gemessen und die Standardabweichung gemäß der nachfolgenden Gleichung ermittelt:

$$Standardabweichung: \quad Cv = \left[\frac{\sum\left(\frac{x_i - \bar{x}}{\bar{x}}\right)^2}{n-1}\right]^{1/2} \times 100[\%]$$

Darin bedeuten: $x_i$ der gemessene Flüssigkeitsvolumenstrom pro Rohr, $\bar{x}$ das arithmetische Mittel der gemessenen Flüssigkeitsvolumenströme über alle $n$ Rohre und $n$ die Anzahl der Rohre

Der Verteiler wurde in allen Versuchen mit einem Flüssigkeitsstand von 50 mm auf dem Rohrboden und unter Einsatz von Rohrtüllen mit tangentialen Einlaufschlitzen (Rechteckschlitze mit 5 mm Schlitzbreite) betrieben.

**Beispiel 1 (Flüssigkeitsverteilung mit und ohne Spontanverdampfung** ohne **Drallbrecher (600); Vergleich):**

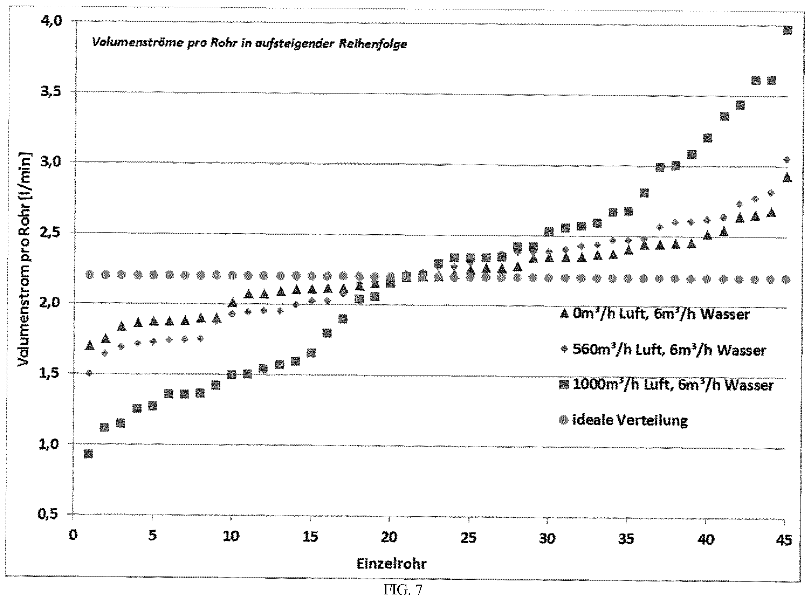

Ein Drallbrecher (600) wurde nicht eingesetzt. Im äußeren Ringspalt (400) kam ein Drallbrecher (700) mit durchgehendem Schlitz für die Flüssigkeit zum Einsatz (wie in FIG. 2b dargestellt). In **FIG. 7** sind die gemessenen Volumenströme pro Rohr in aufsteigender Reihenfolge dargestellt. Es wird deutlich, dass die Verteilung mit zunehmendem Gasphasenanteil (entsprechend zunehmender Spontanverdampfung) immer weiter von der optimalen Verteilung abweicht. Bei 1000 m³/h beträgt die relative Standardabweichung 34 %, was für Verteiler im industriellen Maßstab sehr hoch ist. Bei 0 m³/h Luft beträgt die Standardabweichung 12 % und liegt damit im Bereich üblicher Standardabweichungen industrieller Flüssigkeitsverteiler.

Die Versuche zeigen, dass die Gasphase die Verteilung der Flüssigkeit auf die Rohre massiv stört, *obwohl Gas und Flüssigkeit direkt nach Eintritt in die Haube sauber getrennt wurden und die Flüssigkeit durch den Drallbrecher (700) vorverteilt wird.*

**Beispiel 2 (Flüssigkeitsverteilung mit Spontanverdampfung mit Drallbrecher (600) für die Gasphase (11); erfindungsgemäß):**

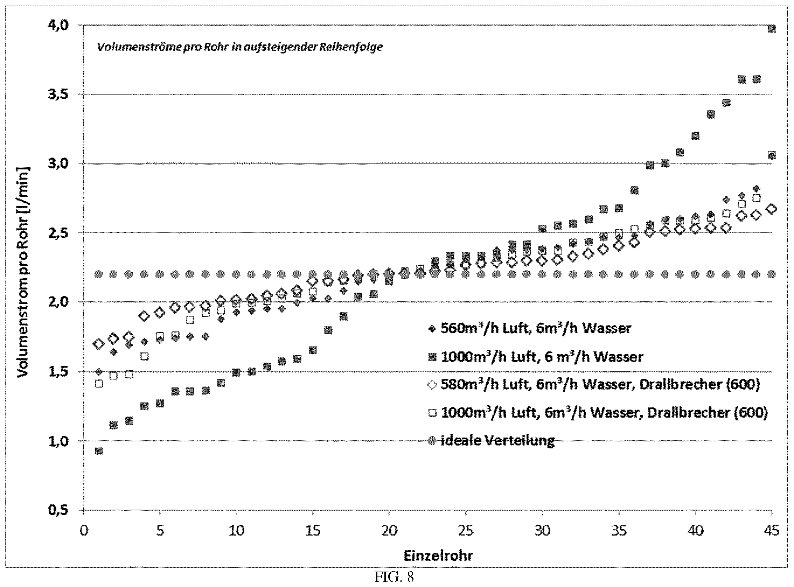

Die Versuche mit 560 m³/h und 1000 m³/h Luft aus Beispiel 1 wurden nach Einbau des Drallbrechers (600) wiederholt. Es zeigt sich damit - siehe **FIG. 8** (zum besseren Vergleich sind die Resultate aus Beispiel 1 mit eingezeichnet) - eine deutliche Verbesserung der Flüssigkeitsverteilung. Bei 1000 m³/h kann die relative Standardabweichung damit von zuvor 34 % auf 16 % reduziert werden. Die damit erzielte Verteilung ist nahe der Verteilung mit 0 m³/h Luft aus Beispiel 1. Das Beispiel zeigt also, dass durch den Einsatz des Drallbrechers (600) der negative Einfluss der Luft auf die Flüssigkeitsverteilung nahezu eliminiert werden kann. Im Beispiel kam ein einfacher Drallbrecher (600) mit 4 Flügeln zum Einsatz, der noch weiter optimiert werden kann (z. B. 8 Flügel, um die Luftströmung (allgemein: die Gasströmung) noch stärker zu beruhigen).

Im erfindungsgemäßen Beispiel 2 konnte die Flüssigkeitsverteilung damit gegenüber Beispiel 1 deutlich verbessert werden, ohne dass die Flüssigkeit auf dem Rohrboden (900) komplett beruhigt wurde. Stattdessen war weiterhin eine deutliche Kreisströmung in der Flüssigkeit sichtbar, welche die Bildung von Ablagerungen bei dazu neigenden Systemen vermeidet.

**Patentansprüche**

1. **Vorrichtung (10000)** geeignet zum gleichmäßigen Aufteilen eines Fluids (10) in $n$ Fluidströme jeweils enthaltend Gas (11) und Flüssigkeit (12), wobei $n$ für eine natürliche Zahl von größer oder gleich 2 steht, wobei die Vorrichtung (10 000) Folgendes umfasst:

   • eine aufrecht angeordnete Haube (100), die unten durch einen Boden (900) begrenzt wird;
   • einen in einer seitlichen Begrenzungswand der Haube (100) angeordneten Eintrittsstutzen (200) für ein Fluid (10), welcher Eintrittsstutzen (200) so ausgestaltet ist, dass eine tangentiale Einströmung des Fluids (10) bewirkt wird;
   • einen im Inneren der Haube aufrecht angeordneten Einbau (300),

   ◦ der mit der Innenwand der Haube (100) einen Ringspalt (400) für nach unten strömende Flüssigkeit (12) bildet,
   ◦ dessen Oberkante oberhalb des Eintrittsstutzens (200) und unterhalb der oberen Begrenzung der Haube (100) endet, sodass die Oberkante des Einbaus (300) und die obere Begrenzung der Haube (100) einen Durchlass (510) für Gas (11) bilden,

◦ der oben mindestens eine Öffnung (310) für einströmendes Gas (11) aufweist,

o dessen Unterkante oberhalb des Bodens (900) endet, sodass die Unterkante des Einbaus (300) und der Boden (900) einen Durchlass (520) für Flüssigkeit (12) bilden und

o der unten mindestens eine Öffnung (320) aufweist, die mit der mindestens einen oberen Öffnung (310) strömungstechnisch verbunden ist;

• n Führungseinrichtungen (800) für n Fluidströme aus Flüssigkeit (12) und Gas (11), welche Führungseinrichtungen (800) durch die Öffnung (320) hindurch in den Einbau (300) ragen und die über Eintrittsöffnungen (810) für Flüssigkeit (12) und über Eintrittsöffnungen (820) für Gas (11) verfügen;

**dadurch gekennzeichnet, dass** im Inneren des Einbaus (300) ein Drallbrecher (600) für nach unten strömendes Gas (11) angeordnet ist.

2. Vorrichtung (10000) gemäß Anspruch 1, bei welcher sich der Querschnitt des Einbaus (300) nach unten erweitert.

3. Vorrichtung (10000) gemäß Anspruch 1 oder 2, bei welcher im Ringspalt (400) unterhalb des Eintrittsstutzens (200) ein Drallbrecher (700) für Flüssigkeit (12) mit Durchtrittsöffnungen (710) für Flüssigkeit (12) angeordnet ist.

4. Vorrichtung (10000) gemäß Anspruch 3, bei welcher der Drallbrecher (700) für Flüssigkeit (12) ein Blech ist, bei welchem die Durchtrittsöffnungen (710) für Flüssigkeit (12) durch mindestens zwei Löcher, die insbesondere über die gesamte Fläche des Blechs gleichmäßig verteilt sind, und/oder durch mindestens einen Schlitz, der insbesondere die gesamte Fläche des Blechs durchläuft, gebildet werden.

5. Vorrichtung gemäß einem der vorstehenden Ansprüche, bei welcher die Öffnung (310) für einströmendes Gas (11) über den gesamten Innenquerschnitt des Einbaus (300) reicht, und/oder bei welcher die Öffnung (320), durch welche die n Führungseinrichtungen (800) in den Einbau (300) ragen, über den gesamten Innenquerschnitt des Einbaus (300) reicht.

6. Vorrichtung gemäß einem der vorstehenden Ansprüche, bei welcher die n Führungseinrichtungen (800) Rohrtüllen oder Bestandteile von Rohren (1 000) sind.

7. Vorrichtung gemäß Anspruch 6, bei welcher die Eintrittsöffnungen (810) für Flüssigkeit (12)

• Tangentialschlitze sind, wobei die Tangentialschlitze insbesondere so angeordnet sind, dass die auf der Innenseite der Führungseinrichtungen (800) erzeugte Strömung der Flüssigkeit (12) die gleiche Richtung wie die durch die Anordnung des Eintrittsstutzens (200) im Ringspalt (400) erzeugte Strömung der Flüssigkeit (12) hat oder

• Axialschlitze

sind.

8. Vorrichtung gemäß einem der vorstehenden Ansprüche, bei welcher der Drallbrecher (600) für nach unten strömendes Gas (11) ausgewählt ist aus der Gruppe bestehend aus Drallkreuzen, Packungen, Drahtgestricken, Lochblechen und Umlenkblechen.

9. Vorrichtung (10000) gemäß einem der vorstehenden Ansprüche, bei welcher die n Führungseinrichtungen (800) bis zu einer Höhe H im Bereich von 10 mm bis 100 mm in den Einbau (300) hineinragen.

10. Fallfilmverdampfer (100000) umfassend

• einen äußeren, umhüllenden Mantel (2000),
• n Rohre (1 000), die in einem oberen und einem unteren Rohrboden befestigt sind, wobei n für eine natürliche Zahl von größer oder gleich 2 steht, an deren Innenseite ein Flüssigkeitsfilm ablaufen kann,
• eine oberseitige Verteilervorrichtung zum Verteilen von Flüssigkeit und Gas in die einzelnen Rohre (1 000),
• eine Zuführeinrichtung (3 100) für ein Heizmedium (20) in den durch die Außenseite der Rohre (1 000) und den Mantel (2000) gebildeten Rohraußenraum,
• eine Abführeinrichtung (3200) für abgekühltes Heizmedium (21) aus dem Rohraußenraum,

• eine Dampfabzugseinrichtung (4 100) für Dampf (13),
• eine Flüssigkeitsabzugseinrichtung (4200) für rohrinnenseitig nicht verdampfte Restflüssigkeit (14),
• eine Vorrichtung (5000) zum Trennen und Sammeln von Restflüssigkeit (14) und Dampf (13),

**dadurch gekennzeichnet, dass** die oberseitige Verteilervorrichtung zum Verteilen von Flüssigkeit und Dampf in die einzelnen Rohre (1 000) eine Vorrichtung (10000) gemäß einem der Ansprüche 1 bis 9 ist, wobei der Boden (900) der Vorrichtung (10000) den oberen Rohrboden des Fallfilmverdampfers (100000) bildet, wobei die *n* Führungseinrichtungen (800) mit den *n* Rohren (1 000) an deren Oberseite verbunden sind oder wobei die *n* Führungseinrichtungen (800) Bestandteile der *n* Rohre (1 000) sind.

11. Fallfilmverdampfer gemäß Anspruch 10, weiterhin umfassend eine Rückführeinrichtung (6000) für die Rückführung eines Teils der Restflüssigkeit (14) in die Verteilervorrichtung (10000), wobei die Rückführeinrichtung (6000)

• eine Mischeinrichtung (6100) zur Vermischung des zurückgeführten Teils der Restflüssigkeit (14) mit dem Fluid (10) vor Eintritt in die Verteilervorrichtung (10000) umfasst, oder
• in einen vom Eintrittsstutzen (200) verschiedenen Eintrittsstutzen (210) mündet, welcher Eintrittsstutzen (210) in einer seitlichen Begrenzungswand der Haube (100) angeordnet ist.

12. Verwendung einer Verteilervorrichtung (10 000) gemäß einem der Ansprüche 1 bis 9 und insbesondere eines Fallfilmverdampfers (100000) gemäß Anspruch 10 oder 11 in der Herstellung und/oder Aufarbeitung eines chemischen Produkts ausgewählt aus der Gruppe bestehend aus organischen Nitroverbindungen, organischen primären Aminen, Isocyanaten, Polyetherpolyolen und Polycarbonaten.

13. Verwendung gemäß Anspruch 12, bei welcher der Fallfilmverdampfer (100 000) in der Aufarbeitung eines Isocyanats zur Verdampfung des Isocyanats eingesetzt wird.

14. Verwendung gemäß Anspruch 13, bei welcher die Verdampfung des Isocyanats zur Abreicherung des Isocyanats aus einem rückstandshaltigen Destillationssumpfstrom der Isocyanatherstellung und/oder zur Trennung einer monomeren Isocyanatfraktion, die verdampft wird, von einer polymeren Isocyanatfraktion eingesetzt wird.

15. Verwendung gemäß einem der Ansprüche 13 oder 14, bei welcher das Isocyanat ausgewählt ist aus der Gruppe bestehend aus Toluylendiisocyanat, den Di- und Polyisocyanate der Diphenylmethanreihe, Hexamethylendiisocyanat, Pentamethlyendiisocyanat, Isophorondiisocyanat, Xylylendiisocyanat, Naphtalindiisocyanat und Dicyclohexylmethandiisocyanat.

**Claims**

1. **Device (10 000)** suitable for uniform division of a fluid (10) into n fluid streams each containing gas (11) and liquid (12), where n is a natural number greater than or equal to 2, where the device (10 000) comprises the following:

• an upright cap (100) which is bounded at the bottom by a plate (900);
• an entry port (200) for a fluid (10) arranged in a lateral delimiting wall of the cap (100), which entry port (200) is configured so that tangential inflow of the fluid (10) is brought about;
• an internal (300) arranged upright in the interior of the cap,

o which together with the interior wall of the cap (100) forms an annular gap (400) for downflowing liquid (12),
o whose upper edge ends above the entry port (200) and below the upper delimitation of the cap (100) so that the upper edge of the internal (300) and the upper delimitation of the cap (100) form a passage (510) for gas (11),
o which at the top has at least one opening (310) for inflowing gas (11),
o whose lower edge ends above the plate (900) so that the lower edge of the internal (300) and the plate (900) form a passage (520) for liquid (12) and
o which at the bottom has at least one opening (320) which is hydrodynamically connected to the at least one upper opening (310);

• n guide devices (800) for n fluid streams composed of liquid (12) and gas (11), which guide devices (800) protrude through the opening (320) into the internal (300) and have entry openings (810) for liquid (12) and entry openings (820) for gas (11);

**characterized in that** a swirl breaker (600) for downward-flowing gas (11) is arranged in the interior of the internal (300).

2. Device (10 000) according to Claim 1, wherein the cross section of the internal (300) widens in a downward direction.

3. Device (10 000) according to Claim 1 or 2, wherein a swirl breaker (700) for liquid (12) having through-openings (710) for liquid (12) is arranged in the annular gap (400) below the entry port (200).

4. Device (10 000) according to Claim 3, wherein the swirl breaker (700) for liquid (12) is a metal plate in which the through-openings (710) for liquid (12) are formed by at least two holes which are in particular distributed uniformly over the entire area of the plate and/or by at least one slit which, in particular, runs through the entire area of the plate.

5. Device according to any of the preceding claims, wherein the opening (310) for inflowing gas (11) extends over the entire internal cross section of the internal (300) and/or the opening (320) through which the n guide devices (800) protrude into the internal (300) extends over the entire internal cross section of the internal (300) .

6. Device according to any of the preceding claims, wherein the n guide devices (800) are tube spouts or constituents of tubes (1000).

7. Device according to Claim 6, wherein the entry openings (810) for liquid (12)

   • are tangential slits which are, in particular, arranged so that the flow of the liquid (12) produced on the inside of the guide devices (800) has the same direction as the flow of the liquid (12) produced in the annular gap (400) by the arrangement of the entry port (200) or
   • are axial slits.

8. Device according to any of the preceding claims, wherein the swirl breaker (600) for downward-flowing gas (11) is selected from the group consisting of swirl crosses, packings, knitted wire meshes, perforated plates and deflection plates.

9. Device (10 000) according to any of the preceding claims, wherein the n guide devices (800) protrude to a height H in the range from 10 mm to 100 mm into the internal (300).

10. Falling film evaporator (100 000) comprising

    • an outer, enclosing shell (2000),
    • n tubes (1000) which are fastened in an upper tube plate and a lower tube plate, where n is a natural number greater than or equal to 2, on the inside of which a liquid film can flow down,
    • an upper distributor device for distributing liquid and gas into the individual tubes (1000),
    • a feed device (3100) for feeding a heating medium (20) into the tube exterior space formed by the outside of the tubes (1000) and the shell (2000),
    • a discharge device (3200) for discharging cooled heating medium (21) from the tube exterior space,
    • a vapor offtake device (4100) for vapor (13),
    • a liquid offtake device (4200) for residual liquid (14) which has not been vaporized inside the tubes,
    • a device (5000) for separating and collecting residual liquid (14) and vapor (13),
    **characterized in that** the upper distributor device for distributing liquid and vapor into the individual tubes (1000) is a device (10 000) according to any of Claims 1 to 9, where the bottom (900) of the device (10 000) forms the upper tube plate of the falling film evaporator (100 000) and the n guide devices (800) are joined to the n tubes (1000) at the upper end thereof or the n guide devices (800) are constituents of the n tubes (1000).

11. Falling film evaporator according to Claim 10 which further comprises a return device (6000) for recirculating part of the residual liquid (14) into the distributor device (10 000), where the return device (6000)

    • comprises a mixing device (6100) for mixing the recirculated part of the residual liquid (14) with the fluid (10) before entry into the distributor device (10 000), or
    • opens into an entry port (210) which is different from the entry port (200) and is arranged in a lateral delimiting wall of the cap (100).

12. Use of a distributor device (10 000) according to any of Claims 1 to 9 and in particular of a falling film evaporator (100 000) according to Claim 10 or 11 in the production and/or work-up of a chemical product selected from the group consisting of organic nitro compounds, organic primary amines, isocyanates, polyether polyols and polycarbonates.

13. Use according to Claim 12, wherein the falling film evaporator (100 000) is used in the work-up of an isocyanate for vaporization of the isocyanate.

14. Use according to Claim 13, wherein the vaporization of the isocyanate is used for at least partially removing the isocyanate from a residue-containing distillation bottoms stream from isocyanate production and/or for separating a monomeric isocyanate fraction, which is vaporized, from a polymeric isocyanate fraction.

15. Use according to either Claim 13 or 14, wherein the isocyanate is selected from the group consisting of tolylene diisocyanate, the diisocyanates and polyisocyanates of the diphenylmethane series, hexamethylene diisocyanate, pentamethylene diisocyanate, isophorone diisocyanate, xylylene diisocyanate, naphthalene diisocyanate and dicyclohexylmethane diisocyanate.

## Revendications

1. **Dispositif (10 000)** convenant à la répartition uniforme d'un fluide (10) en n courants de fluide contenant chacun un gaz (11) et un liquide (12), n représentant un nombre entier supérieur ou égal à 2, le dispositif (10 000) comprenant ce qui suit :

   • une calotte (100), disposée debout, qui est délimitée à sa partie inférieure par un fond (900) ;
   • une tubulure d'entrée (200) pour un fluide (10), disposée dans une paroi de limitation latérale de la calotte (100), la tubulure d'entrée (200) étant conçue de façon à provoquer une entrée tangentielle du fluide (10) ;
   • un ensemble (300), disposé debout dans l'intérieur de la calotte,

      o qui avec la paroi intérieure de la calotte (100) forme un espace annulaire (400) pour un liquide (12) s'écoulant vers le bas,
      o dont l'arête supérieure se termine au-dessus de la tubulure d'entrée (200) et en dessous de la délimitation supérieure de la calotte (100), de sorte que l'arête supérieure de l'ensemble (300) et la délimitation supérieure de la calotte (100) forment un passage (510) pour un gaz (11),
      o qui à sa partie supérieure comprend au moins une ouverture (310) pour un gaz entrant (11),
      o dont l'arête inférieure se termine au-dessus du fond (900), de sorte que l'arête inférieure de l'ensemble (300) et le fond (900) forment un passage (520) pour un liquide (12) et
      o qui présente à sa partie inférieure au moins une ouverture (320), qui est reliée en liaison fluidique avec l'au moins une ouverture supérieure (310) ;

   • n dispositifs de guidage (800) pour n courants de fluide constitués du liquide (12) et du gaz (11), les dispositifs de guidage (800) pénétrant dans l'ensemble (300) en passant par l'ouverture (320), et qui disposent d'ouvertures d'entrée (810) pour le liquide (12) et d'ouvertures d'entrée (820) pour le gaz (11) ;

   **caractérisé en ce qu'**un anti-tourbillon (600) pour un gaz (11) s'écoulant vers le bas est disposé à l'intérieur de l'ensemble (300).

2. Dispositif (10 000) selon la revendication 1, dans lequel la section transversale de l'ensemble (300) s'élargit vers le bas.

3. Dispositif (10 000) selon la revendication 1 ou 2, dans lequel un anti-tourbillon (700) pour le liquide (12), comportant des ouvertures de passage (710) pour le liquide (12), est disposé dans l'espace annulaire (400) en dessous de la tubulure d'entrée (200).

4. Dispositif (10 000) selon la revendication 3, dans lequel l'anti-tourbillon (700) pour le liquide (12) est une tôle, les orifices de passage (710) pour le liquide (12) étant formés par au moins deux trous, qui sont en particulier répartis uniformément sur toute la surface de la tôle, et/ou par au moins une fente qui en particulier court sur toute la surface de la tôle.

5. Dispositif selon l'une des revendications précédentes, dans lequel l'ouverture (310) pour le gaz entrant (11) s'étend sur la totalité de la section transversale intérieure de l'ensemble (300), et/ou dans lequel l'ouverture (320), par laquelle les n dispositifs de guidage (800) dépassent dans l'ensemble (300), s'étend sur la totalité de la section transversale intérieure de l'ensemble (300).

6. Dispositif selon l'une des revendications précédentes, dans lequel les n dispositifs de guidage (800) sont des douilles ou des constituants de tubes (1 000).

7. Dispositif selon la revendication 6, dans lequel les ouvertures d'entrée (810) pour le liquide (12) sont

   • des fentes tangentielles, les fentes tangentielles étant en particulier disposées de telle sorte que l'écoulement de liquide (12), formé sur la face intérieure des dispositifs de guidage (800), écoulement qui a la même direction que l'écoulement du liquide (12) produit par la disposition de la tubulure d'entrée (200) dans l'espace annulaire (400), ou
   • des fentes axiales.

8. Dispositif selon l'une des revendications précédentes, dans lequel l'anti-tourbillon (600) pour le gaz (11) s'écoulant vers le bas est choisi dans le groupe consistant en un croisillon anti-turbulence, des garnissages, des tricotages de fils, des tôles perforées et des chicanes.

9. Dispositif (10 000) selon l'une des revendications précédentes, dans lequel les n dispositifs de guidage (800) dépassent dans l'ensemble (300) jusqu'à une hauteur H dans la plage de 10 mm à 100 mm.

10. Évaporateur à film tombant (100 000) comprenant

    • une enveloppe extérieure entourante (2 000),
    • n tubes (1 000), qui sont fixés dans une plaque tubulaire supérieure et une plaque tubulaire inférieure, n représentant un nombre entier supérieur ou égal à 2, sur la face intérieure de laquelle peut couler un film liquide,
    • un dispositif répartiteur supérieur, destiné à répartir un liquide et un gaz dans les différents tubes (1 000),
    • un dispositif d'amenée (3 100) pour un fluide caloporteur (20) dans l'espace extérieur tubulaire formé par la face extérieure des tubes (1 000) et l'enveloppe (2 000),
    • un dispositif d'évacuation (3 200) pour le fluide caloporteur refroidi (21) à partir de l'espace extérieur tubulaire,
    • un dispositif de soutirage de vapeur (4 100) pour la vapeur (13),
    • un dispositif de soutirage de liquide (4 200) pour le liquide résiduel (14) non évaporé à l'intérieur des tubes,
    • un dispositif (5 000) pour séparer et réunir le liquide résiduel (14) et la vapeur (13),

    **caractérisé en ce que** le dispositif répartiteur supérieur destiné à répartir le liquide et la vapeur dans les différents tubes (1 000) est un dispositif (10 000) selon l'une des revendications 1 à 9, le fond (900) du dispositif (10 000) formant la plaque tubulaire supérieure de l'évaporateur à film tombant (100 000), les n dispositifs de guidage (800) étant reliés aux n tubes (1 000) sur leur face supérieure, et les n dispositifs de guidage (800) étant des constituants des n tubes (1 000).

11. Évaporateur à film tombant selon la revendication 10, comprenant en outre un dispositif de retour (6 000) pour le retour d'une partie du liquide résiduel (14) dans le dispositif répartiteur (10 000), le dispositif de retour (6 000)

    • comprenant un dispositif mélangeur (6 100) pour mélanger la partie renvoyée du liquide résiduel (14) avec le fluide (10) avant entrée dans le dispositif répartiteur (10 000), ou
    • débouchant dans une tubulure d'entrée (210) différente de la tubulure d'entrée (200), la tubulure d'entrée (210) étant disposée dans une paroi de séparation latérale de la calotte (100).

12. Utilisation d'un dispositif répartiteur (10 000) selon l'une des revendications 1 à 9 et en particulier d'un évaporateur à film tombant (100 000) selon la revendication 10 ou 11 pour la fabrication et/ou le traitement d'un produit chimique choisi dans le groupe consistant en les composés nitrés organiques, les amines primaires organiques, les isocyanates, les polyétherpolyols et les polycarbonates.

**13.** Utilisation selon la revendication 12, l'évaporateur à film tombant (100 000), lors du traitement d'un isocyanate, est utilisé pour l'évaporation de l'isocyanate.

**14.** Utilisation selon la revendication 13, l'évaporation de l'isocyanate étant utilisée pour appauvrir en isocyanate le courant de fond, contenant des résidus, de la distillation de la fabrication des isocyanates et/ou pour séparer une fraction isocyanate monomère, qui subit une évaporation, d'une fraction isocyanate polymère.

**15.** Utilisation selon l'une des revendications 13 ou 14, l'isocyanate étant choisi dans le groupe consistant en le diisocyanate de toluylène, les di- et polyisocyanates de la série du diphénylméthane, le diisocyanate d'hexaméthylène, le diisocyanate de pentaméthylène, le diisocyanate d'isophorone, le diisocyanate de xylylène, le diisocyanate de naphtalène et le diisocyanate de dicyclohexylméthane.

FIG. 1

FIG. 2a

FIG. 2b

FIG. 3

FIG. 4

FIG. 5

FIG. 6a

FIG. 6b

Volumenströme pro Rohr in aufsteigender Reihenfolge

▲ 0m³/h Luft, 6m³/h Wasser
◆ 560m³/h Luft, 6m³/h Wasser
■ 1000m³/h Luft, 6m³/h Wasser
● ideale Verteilung

Volumenstrom pro Rohr [l/min]

Einzelrohr

FIG. 7

FIG. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 103980159 A
- US 6066232 A
- DE 29520263 U
- US 3849232 A
- CN 204159052 U
- CN 203342399 U
- US 4094734 A
- DE 1126358 B
- CN 203425542 U

- CH 385169 A
- DE 2103889 A1
- DE 1992031 U
- DD 279613 A
- DE 2604389
- US 4154642 A
- US 4199537 A
- DE 1769607
- GB 1209119 A


**In der Beschreibung aufgeführte Nicht-Patentliteratur**

- **SCHOLL, RINNER.** Verdampfung und Kondensation in Fluidverfahrenstechnik. Wiley, 2006

- Piping. **I. SUTTON.** Vortex breakers prior to installion in Plant Design and Operations. Elsevier, 2017, 162